# EUROPEAN PATENT APPLICATION

(11) **EP 1 782 821 A1**
(43) Date of publication of application: **09.05.2007**
(21) Application number: 05767287.5
(22) Date of filing: 29.07.2005
(51) Int. Cl.: A61K 38/00, A61K 48/00, A61P 9/10, A61P 25/28

(54) **DRUG AND METHOD FOR IMPROVING BRAIN FUNCTION**

(30) Priority: 29.07.2004 JP 2004222649
(71) Applicant: AnGes MG, Inc., Ibaraki-shi, Osaka 567-0085 (JP)
(72) Inventor: KANEDA, Yasufumi, 5620031 (JP); MORISHITA, Ryuichi, 5650851 (JP); SHIMAMURA, Munehisa, Mino-shi, Osaka 5620031 (JP)
(74) Representative: Bates, Philip Ian
(86) International application number: PCT/JP2005/013949
(87) International publication number: WO 2006/011600

(57) **Abstract**

It is intended to provide a novel remedy for improving the brain function or preventing the same from worsening and a novel administration method for the remedy. Namely, a composition for preventing the brain function from worsening or improving the brain function which contains a cell growth factor. It is preferred that this cell frowth factor is one selected from the group consisting of vascular endothelial growth factors (VEGFs), fibroblast growth factors (FGFs) and hepatocyte growth factors (HGFs). A method for preventing the brain function from worsening or improving the brain function which comprises the step of administering a cell growth factor to a patient.

SEQUENCE LISTING

<110> AnGes MG, Inc.
KANEDA, Yasufumi
MORISHITA, Ryuichi
SHIMAMURA, Munehisa

<120> MEDICAMENT AND METHOD FOR AMELIORATIONG BRAIN FUNCTION

<130> AN016PCT

<140> JP 2004-222649
<141> 2004-07-29

<160> 8

<170> PatentIn version 3.2

<210> 1
<211> 2187
<212> DNA
<213> Homo Sapiens

<220>
<221> CDS
<222> (1)..(2187)

<400> 1
atg tgg gtg acc aaa ctc ctg cca gcc ctg ctg ctg cag cat gtc ctc 48
Met Trp Val Thr Lys Leu Leu Pro Ala Leu Leu Leu Gln His Val Leu
1               5                   10                  15

ctg cat ctc ctc ctg ctc ccc atc gcc atc ccc tat gca gag gga caa 96
Leu His Leu Leu Leu Leu Pro Ile Ala Ile Pro Tyr Ala Glu Gly Gln
            20                  25                  30

agg aaa aga aga aat aca att cat gaa ttc aaa aaa tca gca aag act 144
Arg Lys Arg Arg Asn Thr Ile His Glu Phe Lys Lys Ser Ala Lys Thr
        35                  40                  45

acc cta atc aaa ata gat cca gca ctg aag ata aaa acc aaa aaa gtg 192
Thr Leu Ile Lys Ile Asp Pro Ala Leu Lys Ile Lys Thr Lys Lys Val
    50                  55                  60

aat act gca gac caa tgt gct aat aga tgt act agg aat aaa gga ctt 240
Asn Thr Ala Asp Gln Cys Ala Asn Arg Cys Thr Arg Asn Lys Gly Leu
65                  70                  75                  80

cca ttc act tgc aag gct ttt gtt ttt gat aaa gca aga aaa caa tgc 288
Pro Phe Thr Cys Lys Ala Phe Val Phe Asp Lys Ala Arg Lys Gln Cys
                85                  90                  95

ctc tgg ttc ccc ttc aat agc atg tca agt gga gtg aaa aaa gaa ttt 336
Leu Trp Phe Pro Phe Asn Ser Met Ser Ser Gly Val Lys Lys Glu Phe
            100                 105                 110

ggc cat gaa ttt gac ctc tat gaa aac aaa gac tac att aga aac tgc 384
Gly His Glu Phe Asp Leu Tyr Glu Asn Lys Asp Tyr Ile Arg Asn Cys
        115                 120                 125

atc att ggt aaa gga cgc agc tac aag gga aca gta tct atc act aag 432
Ile Ile Gly Lys Gly Arg Ser Tyr Lys Gly Thr Val Ser Ile Thr Lys
    130                 135                 140

agt ggc atc aaa tgt cag ccc tgg agt tcc atg ata cca cac gaa cac 480
Ser Gly Ile Lys Cys Gln Pro Trp Ser Ser Met Ile Pro His Glu His
145                 150                 155                 160

agc ttt ttg cct tcg agc tat cgg ggt aaa gac cta cag gaa aac tac     528
Ser Phe Leu Pro Ser Ser Tyr Arg Gly Lys Asp Leu Gln Glu Asn Tyr
                165                 170                 175

tgt cga aat cct cga ggg gaa gaa ggg gga ccc tgg tgt ttc aca agc     576
Cys Arg Asn Pro Arg Gly Glu Glu Gly Gly Pro Trp Cys Phe Thr Ser
            180                 185                 190

aat cca gag gta cgc tac gaa gtc tgt gac att cct cag tgt tca gaa     624
Asn Pro Glu Val Arg Tyr Glu Val Cys Asp Ile Pro Gln Cys Ser Glu
        195                 200                 205

gtt gaa tgc atg acc tgc aat ggg gag agt tat cga ggt ctc atg gat     672
Val Glu Cys Met Thr Cys Asn Gly Glu Ser Tyr Arg Gly Leu Met Asp
    210                 215                 220

cat aca gaa tca ggc aag att tgt cag cgc tgg gat cat cag aca cca     720
His Thr Glu Ser Gly Lys Ile Cys Gln Arg Trp Asp His Gln Thr Pro
225                 230                 235                 240

cac cgg cac aaa ttc ttg cct gaa aga tat ccc gac aag ggc ttt gat     768
His Arg His Lys Phe Leu Pro Glu Arg Tyr Pro Asp Lys Gly Phe Asp
                245                 250                 255

gat aat tat tgc cgc aat ccc gat ggc cag ccg agg cca tgg tgc tat     816
Asp Asn Tyr Cys Arg Asn Pro Asp Gly Gln Pro Arg Pro Trp Cys Tyr
            260                 265                 270

act ctt gac cct cac acc cgc tgg gag tac tgt gca att aaa aca tgc     864
Thr Leu Asp Pro His Thr Arg Trp Glu Tyr Cys Ala Ile Lys Thr Cys
        275                 280                 285

gct gac aat act atg aat gac act gat gtt cct ttg gaa aca act gaa     912
Ala Asp Asn Thr Met Asn Asp Thr Asp Val Pro Leu Glu Thr Thr Glu
    290                 295                 300

tgc atc caa ggt caa gga gaa ggc tac agg ggc act gtc aat acc att     960
Cys Ile Gln Gly Gln Gly Glu Gly Tyr Arg Gly Thr Val Asn Thr Ile
305                 310                 315                 320

tgg aat gga att cca tgt cag cgt tgg gat tct cag tat cct cac gag     1008
Trp Asn Gly Ile Pro Cys Gln Arg Trp Asp Ser Gln Tyr Pro His Glu
                325                 330                 335

cat gac atg act cct gaa aat ttc aag tgc aag gac cta cga gaa aat     1056
His Asp Met Thr Pro Glu Asn Phe Lys Cys Lys Asp Leu Arg Glu Asn
            340                 345                 350

tac tgc cga aat cca gat ggg tct gaa tca ccc tgg tgt ttt acc act     1104
Tyr Cys Arg Asn Pro Asp Gly Ser Glu Ser Pro Trp Cys Phe Thr Thr
        355                 360                 365

gat cca aac atc cga gtt ggc tac tgc tcc caa att cca aac tgt gat     1152
Asp Pro Asn Ile Arg Val Gly Tyr Cys Ser Gln Ile Pro Asn Cys Asp
    370                 375                 380

atg tca cat gga caa gat tgt tat cgt ggg aat ggc aaa aat tat atg     1200
Met Ser His Gly Gln Asp Cys Tyr Arg Gly Asn Gly Lys Asn Tyr Met
385                 390                 395                 400

ggc aac tta tcc caa aca aga tct gga cta aca tgt tca atg tgg gac     1248
Gly Asn Leu Ser Gln Thr Arg Ser Gly Leu Thr Cys Ser Met Trp Asp
                405                 410                 415

aag aac atg gaa gac tta cat cgt cat atc ttc tgg gaa cca gat gca     1296
Lys Asn Met Glu Asp Leu His Arg His Ile Phe Trp Glu Pro Asp Ala
            420                 425                 430

agt aag ctg aat gag aat tac tgc cga aat cca gat gat gat gct cat 1344
Ser Lys Leu Asn Glu Asn Tyr Cys Arg Asn Pro Asp Asp Asp Ala His
        435                 440                 445

gga ccc tgg tgc tac acg gga aat cca ctc att cct tgg gat tat tgc 1392
Gly Pro Trp Cys Tyr Thr Gly Asn Pro Leu Ile Pro Trp Asp Tyr Cys
    450                 455                 460

cct att tct cgt tgt gaa ggt gat acc aca cct aca ata gtc aat tta 1440
Pro Ile Ser Arg Cys Glu Gly Asp Thr Thr Pro Thr Ile Val Asn Leu
465                 470                 475                 480

gac cat ccc gta ata tct tgt gcc aaa acg aaa caa ttg cga gtt gta 1488
Asp His Pro Val Ile Ser Cys Ala Lys Thr Lys Gln Leu Arg Val Val
                485                 490                 495

aat ggg att cca aca cga aca aac ata gga tgg atg gtt agt ttg aga 1536
Asn Gly Ile Pro Thr Arg Thr Asn Ile Gly Trp Met Val Ser Leu Arg
            500                 505                 510

tac aga aat aaa cat atc tgc gga gga tca ttg ata aag gag agt tgg 1584
Tyr Arg Asn Lys His Ile Cys Gly Gly Ser Leu Ile Lys Glu Ser Trp
        515                 520                 525

gtt ctt act gca cga cag tgt ttc cct tct cga gac ttg aaa gat tat 1632
Val Leu Thr Ala Arg Gln Cys Phe Pro Ser Arg Asp Leu Lys Asp Tyr
    530                 535                 540

gaa gct tgg ctt gga att cat gat gtc cac gga aga gga gat gag aaa 1680
Glu Ala Trp Leu Gly Ile His Asp Val His Gly Arg Gly Asp Glu Lys
545                 550                 555                 560

tgc aaa cag gtt ctc aat gtt tcc cag ctg gta tat ggc cct gaa gga 1728
Cys Lys Gln Val Leu Asn Val Ser Gln Leu Val Tyr Gly Pro Glu Gly
                565                 570                 575

tca gat ctg gtt tta atg aag ctt gcc agg cct gct gtc ctg gat gat 1776
Ser Asp Leu Val Leu Met Lys Leu Ala Arg Pro Ala Val Leu Asp Asp
            580                 585                 590

ttt gtt agt acg att gat tta cct aat tat gga tgc aca att cct gaa 1824
Phe Val Ser Thr Ile Asp Leu Pro Asn Tyr Gly Cys Thr Ile Pro Glu
        595                 600                 605

aag acc agt tgc agt gtt tat ggc tgg ggc tac act gga ttg atc aac 1872
Lys Thr Ser Cys Ser Val Tyr Gly Trp Gly Tyr Thr Gly Leu Ile Asn
    610                 615                 620

tat gat ggc cta tta cga gtg gca cat ctc tat ata atg gga aat gag 1920
Tyr Asp Gly Leu Leu Arg Val Ala His Leu Tyr Ile Met Gly Asn Glu
625                 630                 635                 640

aaa tgc agc cag cat cat cga ggg aag gtg act ctg aat gag tct gaa 1968
Lys Cys Ser Gln His His Arg Gly Lys Val Thr Leu Asn Glu Ser Glu
                645                 650                 655

ata tgt gct ggg gct gaa aag att gga tca gga cca tgt gag ggg gat 2016
Ile Cys Ala Gly Ala Glu Lys Ile Gly Ser Gly Pro Cys Glu Gly Asp
            660                 665                 670

tat ggt ggc cca ctt gtt tgt gag caa cat aaa atg aga atg gtt ctt 2064
Tyr Gly Gly Pro Leu Val Cys Glu Gln His Lys Met Arg Met Val Leu
        675                 680                 685

ggt gtc att gtt cct ggt cgt gga tgt gcc att cca aat cgt cct ggt 2112
Gly Val Ile Val Pro Gly Arg Gly Cys Ala Ile Pro Asn Arg Pro Gly
    690                 695                 700

att ttt gtc cga gta gca tat tat gca aaa tgg ata cac aaa att att 2160
Ile Phe Val Arg Val Ala Tyr Tyr Ala Lys Trp Ile His Lys Ile Ile
705                 710                 715                 720

tta aca tat aag gta cca cag tca tag 2187
Leu Thr Tyr Lys Val Pro Gln Ser
                725

<210> 2
<211> 728
<212> PRT
<213> Homo Sapiens

<400> 2

Met Trp Val Thr Lys Leu Leu Pro Ala Leu Leu Leu Gln His Val Leu
1               5                   10                  15

Leu His Leu Leu Leu Leu Pro Ile Ala Ile Pro Tyr Ala Glu Gly Gln
            20                  25                  30

Arg Lys Arg Arg Asn Thr Ile His Glu Phe Lys Lys Ser Ala Lys Thr
        35                  40                  45

Thr Leu Ile Lys Ile Asp Pro Ala Leu Lys Ile Lys Thr Lys Lys Val
    50                  55                  60

Asn Thr Ala Asp Gln Cys Ala Asn Arg Cys Thr Arg Asn Lys Gly Leu
65                  70                  75                  80

Pro Phe Thr Cys Lys Ala Phe Val Phe Asp Lys Ala Arg Lys Gln Cys
                85                  90                  95

Leu Trp Phe Pro Phe Asn Ser Met Ser Ser Gly Val Lys Lys Glu Phe
            100                 105                 110

Gly His Glu Phe Asp Leu Tyr Glu Asn Lys Asp Tyr Ile Arg Asn Cys
        115                 120                 125

Ile Ile Gly Lys Gly Arg Ser Tyr Lys Gly Thr Val Ser Ile Thr Lys
    130                 135                 140

Ser Gly Ile Lys Cys Gln Pro Trp Ser Ser Met Ile Pro His Glu His
145                 150                 155                 160

Ser Phe Leu Pro Ser Ser Tyr Arg Gly Lys Asp Leu Gln Glu Asn Tyr
                165                 170                 175

Cys Arg Asn Pro Arg Gly Glu Glu Gly Gly Pro Trp Cys Phe Thr Ser
            180                 185                 190

Asn Pro Glu Val Arg Tyr Glu Val Cys Asp Ile Pro Gln Cys Ser Glu
        195                 200                 205

Val Glu Cys Met Thr Cys Asn Gly Glu Ser Tyr Arg Gly Leu Met Asp
    210                 215                 220

His Thr Glu Ser Gly Lys Ile Cys Gln Arg Trp Asp His Gln Thr Pro
225                 230                 235                 240

His Arg His Lys Phe Leu Pro Glu Arg Tyr Pro Asp Lys Gly Phe Asp
                245                 250                 255

Asp Asn Tyr Cys Arg Asn Pro Asp Gly Gln Pro Arg Pro Trp Cys Tyr
            260                 265                 270

Thr Leu Asp Pro His Thr Arg Trp Glu Tyr Cys Ala Ile Lys Thr Cys
        275                 280                 285

Ala Asp Asn Thr Met Asn Asp Thr Asp Val Pro Leu Glu Thr Thr Glu
    290                 295                 300

Cys Ile Gln Gly Gln Gly Glu Gly Tyr Arg Gly Thr Val Asn Thr Ile
305                 310                 315                 320

Trp Asn Gly Ile Pro Cys Gln Arg Trp Asp Ser Gln Tyr Pro His Glu
                325                 330                 335

His Asp Met Thr Pro Glu Asn Phe Lys Cys Lys Asp Leu Arg Glu Asn
            340                 345                 350

Tyr Cys Arg Asn Pro Asp Gly Ser Glu Ser Pro Trp Cys Phe Thr Thr
        355                 360                 365

Asp Pro Asn Ile Arg Val Gly Tyr Cys Ser Gln Ile Pro Asn Cys Asp
    370                 375                 380

Met Ser His Gly Gln Asp Cys Tyr Arg Gly Asn Gly Lys Asn Tyr Met
385                 390                 395                 400

Gly Asn Leu Ser Gln Thr Arg Ser Gly Leu Thr Cys Ser Met Trp Asp
                405                 410                 415

Lys Asn Met Glu Asp Leu His Arg His Ile Phe Trp Glu Pro Asp Ala
            420                 425                 430

Ser Lys Leu Asn Glu Asn Tyr Cys Arg Asn Pro Asp Asp Asp Ala His
        435                 440                 445

Gly Pro Trp Cys Tyr Thr Gly Asn Pro Leu Ile Pro Trp Asp Tyr Cys
    450                 455                 460

Pro Ile Ser Arg Cys Glu Gly Asp Thr Thr Pro Thr Ile Val Asn Leu
465                 470                 475                 480

Asp His Pro Val Ile Ser Cys Ala Lys Thr Lys Gln Leu Arg Val Val
                485                 490                 495

Asn Gly Ile Pro Thr Arg Thr Asn Ile Gly Trp Met Val Ser Leu Arg
            500                 505                 510

Tyr Arg Asn Lys His Ile Cys Gly Gly Ser Leu Ile Lys Glu Ser Trp
        515                 520                 525

Val Leu Thr Ala Arg Gln Cys Phe Pro Ser Arg Asp Leu Lys Asp Tyr
    530                 535                 540

Glu Ala Trp Leu Gly Ile His Asp Val His Gly Arg Gly Asp Glu Lys
545                 550                 555                 560

Cys Lys Gln Val Leu Asn Val Ser Gln Leu Val Tyr Gly Pro Glu Gly
                565                 570                 575

Ser Asp Leu Val Leu Met Lys Leu Ala Arg Pro Ala Val Leu Asp Asp
            580                 585                 590

Phe Val Ser Thr Ile Asp Leu Pro Asn Tyr Gly Cys Thr Ile Pro Glu
        595                 600                 605

Lys Thr Ser Cys Ser Val Tyr Gly Trp Gly Tyr Thr Gly Leu Ile Asn
    610                 615                 620

Tyr Asp Gly Leu Leu Arg Val Ala His Leu Tyr Ile Met Gly Asn Glu
625                 630                 635                 640

Lys Cys Ser Gln His His Arg Gly Lys Val Thr Leu Asn Glu Ser Glu
                645                 650                 655

Ile Cys Ala Gly Ala Glu Lys Ile Gly Ser Gly Pro Cys Glu Gly Asp
            660                 665                 670

Tyr Gly Gly Pro Leu Val Cys Glu Gln His Lys Met Arg Met Val Leu
        675                 680                 685

Gly Val Ile Val Pro Gly Arg Gly Cys Ala Ile Pro Asn Arg Pro Gly
    690                 695                 700

Ile Phe Val Arg Val Ala Tyr Tyr Ala Lys Trp Ile His Lys Ile Ile
705                 710                 715                 720

Leu Thr Tyr Lys Val Pro Gln Ser
                725

<210>  3
<211>  576
<212>  DNA

<213>  Homo sapiens

<220>
<221>  CDS
<222>  (1)..(576)

<400>  3
atg aac ttt ctg ctg tct tgg gtg cat tgg agc ctt gcc ttg ctg ctc      48
Met Asn Phe Leu Leu Ser Trp Val His Trp Ser Leu Ala Leu Leu Leu
1               5                   10                  15

tac ctc cac cat gcc aag tgg tcc cag gct gca ccc atg gca gaa gga      96
Tyr Leu His His Ala Lys Trp Ser Gln Ala Ala Pro Met Ala Glu Gly
            20                  25                  30

gga ggg cag aat cat cac gaa gtg gtg aag ttc atg gat gtc tat cag      144
Gly Gly Gln Asn His His Glu Val Val Lys Phe Met Asp Val Tyr Gln
        35                  40                  45

cgc agc tac tgc cat cca atc gag acc ctg gtg gac atc ttc cag gag      192
Arg Ser Tyr Cys His Pro Ile Glu Thr Leu Val Asp Ile Phe Gln Glu
    50                  55                  60

tac cct gat gag atc gag tac atc ttc aag cca tcc tgt gtg ccc ctg      240
Tyr Pro Asp Glu Ile Glu Tyr Ile Phe Lys Pro Ser Cys Val Pro Leu
65                  70                  75                  80

atg cga tgc ggg ggc tgc tgc aat gac gag ggc ctg gag tgt gtg ccc      288
Met Arg Cys Gly Gly Cys Cys Asn Asp Glu Gly Leu Glu Cys Val Pro
                85                  90                  95

act gag gag tcc aac atc acc atg cag att atg cgg atc aaa cct cac      336
Thr Glu Glu Ser Asn Ile Thr Met Gln Ile Met Arg Ile Lys Pro His
            100                 105                 110

caa ggc cag cac ata gga gag atg agc ttc cta cag cac aac aaa tgt      384
Gln Gly Gln His Ile Gly Glu Met Ser Phe Leu Gln His Asn Lys Cys
        115                 120                 125

gaa tgc aga cca aag aaa gat aga gca aga caa gaa aat ccc tgt ggg      432
Glu Cys Arg Pro Lys Lys Asp Arg Ala Arg Gln Glu Asn Pro Cys Gly
    130                 135                 140

cct tgc tca gag cgg aga aag cat ttg ttt gta caa gat ccg cag acg      480
Pro Cys Ser Glu Arg Arg Lys His Leu Phe Val Gln Asp Pro Gln Thr
145                 150                 155                 160

tgt aaa tgt tcc tgc aaa aac aca gac tcg cgt tgc aag gcg agg cag      528
Cys Lys Cys Ser Cys Lys Asn Thr Asp Ser Arg Cys Lys Ala Arg Gln
                165                 170                 175

ctt gag tta aac gaa cgt act tgc aga tgt gac aag ccg agg cgg tga      576
Leu Glu Leu Asn Glu Arg Thr Cys Arg Cys Asp Lys Pro Arg Arg
            180                 185                 190


<210>  4
<211>  191
<212>  PRT
<213>  Homo sapiens

<400>  4

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Asn Pro Cys Gly
    130                 135                 140

Pro Cys Ser Glu Arg Arg Lys His Leu Phe Val Gln Asp Pro Gln Thr
145                 150                 155                 160

Cys Lys Cys Ser Cys Lys Asn Thr Asp Ser Arg Cys Lys Ala Arg Gln
                165                 170                 175

Leu Glu Leu Asn Glu Arg Thr Cys Arg Cys Asp Lys Pro Arg Arg
            180                 185                 190

<210>  5
<211>  1073
<212>  DNA
<213>  Homo sapiens

<220>
<221>  CDS
<222>  (91)..(558)

<400>  5
ccacgcgtcc gaaaaaatac tgttggcagc agcacaatgt ttgggctaag acctggtctt      60

gaaagcgcca caagcagcag ctgctgagcc atg gct gaa ggg gaa atc acc acc     114
                                 Met Ala Glu Gly Glu Ile Thr Thr
                                 1               5

ttc aca gcc ctg acc gag aag ttt aat ctg cct cca ggg aat tac aag      162
Phe Thr Ala Leu Thr Glu Lys Phe Asn Leu Pro Pro Gly Asn Tyr Lys
    10                  15                  20

aag ccc aaa ctc ctc tac tgt agc aac ggg ggc cac ttc ctg agg atc      210
Lys Pro Lys Leu Leu Tyr Cys Ser Asn Gly Gly His Phe Leu Arg Ile
25                  30                  35                  40

ctt ccg gat ggc aca gtg gat ggg aca agg gac agg agc gac cag cac     258
Leu Pro Asp Gly Thr Val Asp Gly Thr Arg Asp Arg Ser Asp Gln His
            45                  50                  55

att cag ctg cag ctc agt gcg gaa agc gtg ggg gag gtg tat ata aag     306
Ile Gln Leu Gln Leu Ser Ala Glu Ser Val Gly Glu Val Tyr Ile Lys
        60                  65                  70

agt acc gag act ggc cag tac ttg gcc atg gac acc gac ggg ctt tta     354
Ser Thr Glu Thr Gly Gln Tyr Leu Ala Met Asp Thr Asp Gly Leu Leu
    75                  80                  85

tac ggc tca cag aca cca aat gag gaa tgt ttg ttc ctg gaa agg ctg     402
Tyr Gly Ser Gln Thr Pro Asn Glu Glu Cys Leu Phe Leu Glu Arg Leu
90                  95                  100

gag gag aac cat tac aac acc tat ata tcc aag aag cat gca gag aag     450
Glu Glu Asn His Tyr Asn Thr Tyr Ile Ser Lys Lys His Ala Glu Lys
105                 110                 115                 120

aat tgg ttt gtt ggc ctc aag aag aat ggg agc tgc aaa cgc ggt cct     498
Asn Trp Phe Val Gly Leu Lys Lys Asn Gly Ser Cys Lys Arg Gly Pro
                125                 130                 135

cgg act cac tat ggc cag aaa gca atc ttg ttt ctc ccc ctg cca gtc     546
Arg Thr His Tyr Gly Gln Lys Ala Ile Leu Phe Leu Pro Leu Pro Val
            140                 145                 150

tct tct gat taa agagatctgt tctgggtgtt gaccactcca gagaagtttc     598
Ser Ser Asp
        155

gaggggtcct cacctggttg acccaaaaat gttcccttga ccattggctg cgctaacccc     658

cagcccacag agcctgaatt tgtaagcaac ttgcttctaa atgcccagtt cacttctttg     718

cagagccttt tacccctgca cagtttagaa cagagggacc aaattgcttc taggagtcaa     778

ctggctggcc agtctgggtc tgggtttgga tctccaattg cctcttgcag gctgagtccc     838

tcaatgcaaa agtggggcta aatgaagtgt gttaaggggt cagctaagtg ggacattagt     898

aactgcacac tatttccctc tactgagtaa accctatctg tgattccccc aaaaaaaaaa     958

aaaaaaaaaa aaaaaaaaaa aaaaaaaaaa aaaaaaaaaa aaaaaaaaaa aaaaaaaaaa    1018

aaaaaaaaaa aaaaaaaaaa aaaaaaaaaa aaaaaaaaaa aaaaaaaaaa aaaaa    1073

<210>  6
<211>  155
<212>  PRT
<213>  Homo sapiens

<400>  6

Met Ala Glu Gly Glu Ile Thr Thr Phe Thr Ala Leu Thr Glu Lys Phe
1               5                   10                  15

Asn Leu Pro Pro Gly Asn Tyr Lys Lys Pro Lys Leu Leu Tyr Cys Ser
            20                  25                  30

Asn Gly Gly His Phe Leu Arg Ile Leu Pro Asp Gly Thr Val Asp Gly
        35                  40                  45

Thr Arg Asp Arg Ser Asp Gln His Ile Gln Leu Gln Leu Ser Ala Glu
    50                  55                  60

Ser Val Gly Glu Val Tyr Ile Lys Ser Thr Glu Thr Gly Gln Tyr Leu
65                  70                  75                  80

Ala Met Asp Thr Asp Gly Leu Leu Tyr Gly Ser Gln Thr Pro Asn Glu
                85                  90                  95

Glu Cys Leu Phe Leu Glu Arg Leu Glu Glu Asn His Tyr Asn Thr Tyr
            100                 105                 110

Ile Ser Lys Lys His Ala Glu Lys Asn Trp Phe Val Gly Leu Lys Lys
        115                 120                 125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155

<210>  7
<211>  7681
<212>  DNA
<213>  Artificial

<220>
<223>  pcDNA3.1(-)HGF

<400>  7
gacggatcgg gagatctccc gatcccctat ggtgcactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggag tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg     780

gtaggcgtgt acggtgggag gtctatataa gcagagctct ctggctaact agagaaccca     840

ctgcttactg gcttatcgaa attaatacga ctcactatag ggagacccaa gctggctagc     900

gtttaaacgg gccctctaga ctcgagcggc cgctctagaa ctagctggat cctgccagcc 960
cgtccagcag caccatgtgg gtgaccaaac tcctgccagc cctgctgctg cagcatgtcc 1020
tcctgcatct cctcctgctc cccatcgcca tcccctatgc agagggacaa aggaaaagaa 1080
gaaatacaat tcatgaattc aaaaaatcag caaagactac cctaatcaaa atagatccag 1140
cactgaagat aaaaaccaaa aaagtgaata ctgcagacca atgtgctaat agatgtacta 1200
ggaataaagg acttccattc acttgcaagg cttttgtttt tgataaagca agaaaacaat 1260
gcctctggtt ccccttcaat agcatgtcaa gtggagtgaa aaaagaattt ggccatgaat 1320
ttgacctcta tgaaaacaaa gactacatta gaaactgcat cattggtaaa ggacgcagct 1380
acaagggaac agtatctatc actaagagtg gcatcaaatg tcagccctgg agttccatga 1440
taccacacga acacagcttt ttgccttcga gctatcgggg taaagaccta caggaaaact 1500
actgtcgaaa tcctcgaggg gaagaagggg gaccctggtg tttcacaagc aatccagagg 1560
tacgctacga agtctgtgac attcctcagt gttcagaagt tgaatgcatg acctgcaatg 1620
gggagagtta tcgaggtctc atggatcata cagaatcagg caagatttgt cagcgctggg 1680
atcatcagac accacaccgg cacaaattct tgcctgaaag atatcccgac aagggctttg 1740
atgataatta ttgccgcaat cccgatggcc agccgaggcc atggtgctat actcttgacc 1800
ctcacacccg ctgggagtac tgtgcaatta aaacatgcgc tgacaatact atgaatgaca 1860
ctgatgttcc tttggaaaca actgaatgca tccaaggtca aggagaaggc tacaggggca 1920
ctgtcaatac catttggaat ggaattccat gtcagcgttg ggattctcag tatcctcacg 1980
agcatgacat gactcctgaa aatttcaagt gcaaggacct acgagaaaat tactgccgaa 2040
atccagatgg gtctgaatca ccctggtgtt ttaccactga tccaaacatc cgagttggct 2100
actgctccca aattccaaac tgtgatatgt cacatggaca agattgttat cgtgggaatg 2160
gcaaaaatta tatgggcaac ttatcccaaa caagatctgg actaacatgt tcaatgtggg 2220
acaagaacat ggaagactta catcgtcata tcttctggga accagatgca agtaagctga 2280
atgagaatta ctgccgaaat ccagatgatg atgctcatgg accctggtgc tacacgggaa 2340
atccactcat tccttgggat tattgcccta tttctcgttg tgaaggtgat accacaccta 2400
caatagtcaa tttagaccat cccgtaatat cttgtgccaa aacgaaacaa ttgcgagttg 2460
taaatgggat tccaacacga acaaacatag gatggatggt tagtttgaga tacagaaata 2520
aacatatctg cggaggatca ttgataaagg agagttgggt tcttactgca cgacagtgtt 2580
tcccttctcg agacttgaaa gattatgaag cttggcttgg aattcatgat gtccacggaa 2640
gaggagatga gaaatgcaaa caggttctca atgtttccca gctggtatat ggccctgaag 2700
gatcagatct ggttttaatg aagcttgcca ggcctgctgt cdtggatgat tttgttagta 2760
cgattgattt acctaattat ggatgcacaa ttcctgaaaa gaccagttgc agtgtttatg 2820
gctggggcta cactggattg atcaactatg atggcctatt acgagtggca catctctata 2880
taatgggaaa tgagaaatgc agccagcatc atcgagggaa ggtgactctg aatgagtctg 2940

aaatatgtgc tggggctgaa aagattggat caggaccatg tgagggggat tatggtggcc 3000
cacttgtttg tgagcaacat aaaatgagaa tggttcttgg tgtcattgtt cctggtcgtg 3060
gatgtgccat tccaaatcgt cctggtattt ttgtccgagt agcatattat gcaaaatgga 3120
tacacaaaat tattttaaca tataaggtac cacagtcata gctgttaacc cgggtcgaag 3180
cggccgccac tgtgctggat atctgcagaa ttccaccaca ctggactagt ggatccgagc 3240
tcggtaccaa gcttaagttt aaaccgctga tcagcctcga ctgtgccttc tagttgccag 3300
ccatctgttg tttgcccctc ccccgtgcct tccttgaccc tggaaggtgc cactcccact 3360
gtcctttcct aataaaatga ggaaattgca tcgcattgtc tgagtaggtg tcattctatt 3420
ctggggggtg gggtggggca ggacagcaag ggggaggatt gggaagacaa tagcaggcat 3480
gctggggatg cggtgggctc tatggcttct gaggcggaaa gaaccagctg gggctctagg 3540
gggtatcccc acgcgccctg tagcggcgca ttaagcgcgg cgggtgtggt ggttacgcgc 3600
agcgtgaccg ctacacttgc cagcgcccta gcgcccgctc ctttcgcttt cttcccttcc 3660
tttctcgcca cgttcgccgg ctttccccgt caagctctaa atcgggggct ccctttaggg 3720
ttccgattta gtgctttacg gcacctcgac cccaaaaaac ttgattaggg tgatggttca 3780
cgtagtgggc catcgccctg atagacggtt tttcgccctt tgacgttgga gtccacgttc 3840
tttaatagtg gactcttgtt ccaaactgga acaacactca accctatctc ggtctattct 3900
tttgatttat aagggatttt gccgatttcg gcctattggt taaaaaatga gctgatttaa 3960
caaaaattta acgcgaatta attctgtgga atgtgtgtca gttagggtgt ggaaagtccc 4020
caggctcccc agcaggcaga agtatgcaaa gcatgcatct caattagtca gcaaccaggt 4080
gtggaaagtc cccaggctcc ccagcaggca gaagtatgca aagcatgcat ctcaattagt 4140
cagcaaccat agtcccgccc ctaactccgc ccatcccgcc cctaactccg cccagttccg 4200
cccattctcc gccccatggc tgactaattt tttttattta tgcagaggcc gaggccgcct 4260
ctgcctctga gctattccag aagtagtgag gaggcttttt tggaggccta ggcttttgca 4320
aaaagctccc gggagcttgt atatccattt tcggatctga tcaagagaca ggatgaggat 4380
cgtttcgcat gattgaacaa gatggattgc acgcaggttc tccggccgct tgggtggaga 4440
ggctattcgg ctatgactgg gcacaacaga caatcggctg ctctgatgcc gccgtgttcc 4500
ggctgtcagc gcaggggcgc ccggttcttt ttgtcaagac cgacctgtcc ggtgccctga 4560
atgaactgca ggacgaggca gcgcggctat cgtggctggc cacgacgggc gttccttgcg 4620
cagctgtgct cgacgttgtc actgaagcgg gaagggactg gctgctattg ggcgaagtgc 4680
cggggcagga tctcctgtca tctcaccttg ctcctgccga gaaagtatcc atcatggctg 4740
atgcaatgcg gcggctgcat acgcttgatc cggctacctg cccattcgac caccaagcga 4800
aacatcgcat cgagcgagca cgtactcgga tggaagccgg tcttgtcgat caggatgatc 4860
tggacgaaga gcatcagggg ctcgcgccag ccgaactgtt cgccaggctc aaggcgcgca 4920
tgcccgacgg cgaggatctc gtcgtgaccc atggcgatgc ctgcttgccg aatatcatgg 4980

tggaaaatgg ccgcttttct ggattcatcg actgtggccg gctgggtgtg gcggaccgct 5040
atcaggacat agcgttggct acccgtgata ttgctgaaga gcttggcggc gaatgggctg 5100
accgcttcct cgtgctttac ggtatcgccg ctcccgattc gcagcgcatc gccttctatc 5160
gccttcttga cgagttcttc tgagcgggac tctggggttc gaaatgaccg accaagcgac 5220
gcccaacctg ccatcacgag atttcgattc caccgccgcc ttctatgaaa ggttgggctt 5280
cggaatcgtt ttccgggacg ccggctggat gatcctccag cgcggggatc tcatgctgga 5340
gttcttcgcc caccccaact tgtttattgc agcttataat ggttacaaat aaagcaatag 5400
catcacaaat ttcacaaata aagcattttt ttcactgcat tctagttgtg gtttgtccaa 5460
actcatcaat gtatcttatc atgtctgtat accgtcgacc tctagctaga gcttggcgta 5520
atcatggtca tagctgtttc ctgtgtgaaa ttgttatccg ctcacaattc cacacaacat 5580
acgagccgga agcataaagt gtaaagcctg gggtgcctaa tgagtgagct aactcacatt 5640
aattgcgttg cgctcactgc ccgctttcca gtcgggaaac ctgtcgtgcc agctgcatta 5700
atgaatcggc caacgcgcgg ggagaggcgg tttgcgtatt gggcgctctt ccgcttcctc 5760
gctcactgac tcgctgcgct cggtcgttcg gctgcggcga gcggtatcag ctcactcaaa 5820
ggcggtaata cggttatcca cagaatcagg ggataacgca ggaaagaaca tgtgagcaaa 5880
aggccagcaa aaggccagga accgtaaaaa ggccgcgttg ctggcgtttt tccataggct 5940
ccgcccccct gacgagcatc acaaaaatcg acgctcaagt cagaggtggc gaaacccgac 6000
aggactataa agataccagg cgtttccccc tggaagctcc ctcgtgcgct ctcctgttcc 6060
gaccctgccg cttaccggat acctgtccgc ctttctccct tcgggaagcg tggcgctttc 6120
tcatagctca cgctgtaggt atctcagttc ggtgtaggtc gttcgctcca agctgggctg 6180
tgtgcacgaa ccccccgttc agcccgaccg ctgcgcctta tccggtaact atcgtcttga 6240
gtccaacccg gtaagacacg acttatcgcc actggcagca gccactggta acaggattag 6300
cagagcgagg tatgtaggcg gtgctacaga gttcttgaag tggtggccta actacggcta 6360
cactagaaga acagtatttg gtatctgcgc tctgctgaag ccagttacct tcggaaaaag 6420
agttggtagc tcttgatccg gcaaacaaac caccgctggt agcggttttt ttgtttgcaa 6480
gcagcagatt acgcgcagaa aaaaaggatc tcaagaagat cctttgatct tttctacggg 6540
gtctgacgct cagtggaacg aaaactcacg ttaagggatt ttggtcatga gattatcaaa 6600
aaggatcttc acctagatcc ttttaaatta aaaatgaagt tttaaatcaa tctaaagtat 6660
atatgagtaa acttggtctg acagttacca atgcttaatc agtgaggcac ctatctcagc 6720
gatctgtcta tttcgttcat ccatagttgc ctgactcccc gtcgtgtaga taactacgat 6780
acgggagggc ttaccatctg gccccagtgc tgcaatgata ccgcgagacc cacgctcacc 6840
ggctccagat ttatcagcaa taaaccagcc agccggaagg gccgagcgca gaagtggtcc 6900
tgcaacttta tccgcctcca tccagtctat taattgttgc cgggaagcta gagtaagtag 6960
ttcgccagtt aatagtttgc gcaacgttgt tgccattgct acaggcatcg tggtgtcacg 7020

ctcgtcgttt ggtatggctt cattcagctc cggttcccaa cgatcaaggc gagttacatg 7080
atcccccatg ttgtgcaaaa aagcggttag ctccttcggt cctccgatcg ttgtcagaag 7140
taagttggcc gcagtgttat cactcatggt tatggcagca ctgcataatt ctcttactgt 7200
catgccatcc gtaagatgct tttctgtgac tggtgagtac tcaaccaagt cattctgaga 7260
atagtgtatg cggcgaccga gttgctcttg cccggcgtca atacgggata ataccgcgcc 7320
acatagcaga actttaaaag tgctcatcat tggaaaacgt tcttcggggc gaaaactctc 7380
aaggatctta ccgctgttga gatccagttc gatgtaaccc actcgtgcac ccaactgatc 7440
ttcagcatct tttactttca ccagcgtttc tgggtgagca aaaacaggaa ggcaaaatgc 7500
cgcaaaaaag ggaataaggg cgacacggaa atgttgaata ctcatactct tcctttttca 7560
atattattga agcatttatc agggttattg tctcatgagc ggatacatat ttgaatgtat 7620
ttagaaaaat aaacaaatag gggttccgcg cacatttccc cgaaaagtgc cacctgacgt 7680
c 7681

<210> 8
<211> 5181
<212> DNA
<213> Artificial

<220>
<223> pVAX1HGF/MGB1

<400> 8
gctgcttcgc gatgtacggg ccagatatac gcgttgacat tgattattga ctagttatta 60
atagtaatca attacggggt cattagttca tagcccatat atggagttcc gcgttacata 120
acttacggta aatggcccgc ctggctgacc gcccaacgac ccccgcccat tgacgtcaat 180
aatgacgtat gttcccatag taacgccaat agggactttc cattgacgtc aatgggtgga 240
gtatttacgg taaactgccc acttggcagt acatcaagtg tatcatatgc caagtacgcc 300
ccctattgac gtcaatgacg gtaaatggcc cgcctggcat tatgcccagt acatgacctt 360
atgggacttt cctacttggc agtacatcta cgtattagtc atcgctatta ccatggtgat 420
gcggttttgg cagtacatca atgggcgtgg atagcggttt gactcacggg gatttccaag 480
tctccacccc attgacgtca atgggagttt gttttggcac caaaatcaac gggactttcc 540
aaaatgtcgt aacaactccg ccccattgac gcaaatgggc ggtaggcgtg tacggtggga 600
ggtctatata agcagagctc tctggctaac tagagaaccc actgcttact ggcttatcga 660
aattaatacg actcactata gggagaccca agctggctag cgtttaaact taagcttggt 720
accgagctcg gatccgccag cccgtccagc agcaccatgt gggtgaccaa actcctgcca 780
gccctgctgc tgcagcatgt cctcctgcat ctcctcctgc tccccatcgc catcccctat 840
gcagagggac aaaggaaaag aagaaataca attcatgaat tcaaaaaatc agcaaagact 900
accctaatca aaatagatcc agcactgaag ataaaaacca aaaaagtgaa tactgcagac 960
caatgtgcta atagatgtac taggaataaa ggacttccat tcacttgcaa ggcttttgtt 1020

tttgataaag caagaaaaca atgcctctgg ttccccttca atagcatgtc aagtggagtg 1080
aaaaaagaat ttggccatga atttgacctc tatgaaaaca aagactacat tagaaactgc 1140
atcattggta aaggacgcag ctacaaggga acagtatcta tcactaagag tggcatcaaa 1200
tgtcagccct ggagttccat gataccacac gaacacagct ttttgccttc gagctatcgg 1260
ggtaaagacc tacaggaaaa ctactgtcga aatcctcgag gggaagaagg gggaccctgg 1320
tgtttcacaa gcaatccaga ggtacgctac gaagtctgtg acattcctca gtgttcagaa 1380
gttgaatgca tgacctgcaa tggggagagt tatcgaggtc tcatggatca tacagaatca 1440
ggcaagattt gtcagcgctg ggatcatcag acaccacacc ggcacaaatt cttgcctgaa 1500
agatatcccg acaagggctt tgatgataat tattgccgca atcccgatgg ccagccgagg 1560
ccatggtgct atactcttga ccctcacacc cgctgggagt actgtgcaat taaaacatgc 1620
gctgacaata ctatgaatga cactgatgtt cctttggaaa caactgaatg catccaaggt 1680
caaggagaag gctacagggg cactgtcaat accatttgga atggaattcc atgtcagcgt 1740
tgggattctc agtatcctca cgagcatgac atgactcctg aaaatttcaa gtgcaaggac 1800
ctacgagaaa attactgccg aaatccagat gggtctgaat caccctggtg ttttaccact 1860
gatccaaaca tccgagttgg ctactgctcc caaattccaa actgtgatat gtcacatgga 1920
caagattgtt atcgtgggaa tggcaaaaat tatatgggca acttatccca aacaagatct 1980
ggactaacat gttcaatgtg ggacaagaac atggaagact tacatcgtca tatcttctgg 2040
gaaccagatg caagtaagct gaatgagaat tactgccgaa atccagatga tgatgctcat 2100
ggaccctggt gctacacggg aaatccactc attccttggg attattgccc tatttctcgt 2160
tgtgaaggtg ataccacacc tacaatagtc aatttagacc atcccgtaat atcttgtgcc 2220
aaaacgaaac aattgcgagt tgtaaatggg attccaacac gaacaaacat aggatggatg 2280
gttagtttga gatacagaaa taaacatatc tgcggaggat cattgataaa ggagagttgg 2340
gttcttactg cacgacagtg tttcccttct cgagacttga aagattatga agcttggctt 2400
ggaattcatg atgtccacgg aagaggagat gagaaatgca aacaggttct caatgtttcc 2460
cagctggtat atggccctga aggatcagat ctggttttaa tgaagcttgc caggcctgct 2520
gtcctggatg attttgttag tacgattgat ttacctaatt atggatgcac aattcctgaa 2580
aagaccagtt gcagtgttta tggctggggc tacactggat tgatcaacta tgatggccta 2640
ttacgagtgg cacatctcta tataatggga aatgagaaat gcagccagca tcatcgaggg 2700
aaggtgactc tgaatgagtc tgaaatatgt gctggggctg aaaagattgg atcaggacca 2760
tgtgaggggg attatggtgg cccacttgtt tgtgagcaac ataaaatgag aatggttctt 2820
ggtgtcattg ttcctggtcg tggatgtgcc attccaaatc gtcctggtat ttttgtccga 2880
gtagcatatt atgcaaaatg gatacacaaa attattttaa catataaggt accacagtca 2940
tagctgttaa cccgggtcga agcggccgct cgagtctaga gggcccgttt aaacccgctg 3000
atcagcctcg actgtgcctt ctagttgcca gccatctgtt gtttgcccct cccccgtgcc 3060

ttccttgacc ctggaaggtg ccactcccac tgtcctttcc taataaaatg aggaaattgc 3120
atcgcattgt ctgagtaggt gtcattctat tctggggggt ggggtggggc aggacagcaa 3180
gggggaggat tgggaagaca atagcaggca tgctggggat gcggtgggct ctatggcttc 3240
tactgggcgg ttttatggac agcaagcgaa ccggaattgc cagctggggc gccctctggt 3300
aaggttggga agccctgcaa agtaaactgg atggctttct tgccgccaag gatctgatgg 3360
cgcaggggat caagctctga tcaagagaca ggatgaggat cgtttcgcat gattgaacaa 3420
gatggattgc acgcaggttc tccggccgct tgggtggaga ggctattcgg ctatgactgg 3480
gcacaacaga caatcggctg ctctgatgcc gccgtgttcc ggctgtcagc gcaggggcgc 3540
ccggttcttt ttgtcaagac cgacctgtcc ggtgccctga atgaactgca agacgaggca 3600
gcgcggctat cgtggctggc cacgacgggc gttccttgcg cagctgtgct cgacgttgtc 3660
actgaagcgg gaagggactg gctgctattg ggcgaagtgc cggggcagga tctcctgtca 3720
tctcaccttg ctcctgccga gaaagtatcc atcatggctg atgcaatgcg gcggctgcat 3780
acgcttgatc cggctacctg cccattcgac caccaagcga aacatcgcat cgagcgagca 3840
cgtactcgga tggaagccgg tcttgtcgat caggatgatc tggacgaaga gcatcagggg 3900
ctcgcgccag ccgaactgtt cgccaggctc aaggcgagca tgcccgacgg cgaggatctc 3960
gtcgtgaccc atggcgatgc ctgcttgccg aatatcatgg tggaaaatgg ccgcttttct 4020
ggattcatcg actgtggccg gctgggtgtg gcggaccgct atcaggacat agcgttggct 4080
acccgtgata ttgctgaaga gcttggcggc gaatgggctg accgcttcct cgtgctttac 4140
ggtatcgccg ctcccgattc gcagcgcatc gccttctatc gccttcttga cgagttcttc 4200
tgaattatta acgcttacaa tttcctgatg cggtattttc tccttacgca tctgtgcggt 4260
atttcacacc gcatcaggtg gcacttttcg gggaaatgtg cgcggaaccc ctatttgttt 4320
atttttctaa atacattcaa atatgtatcc gctcatgaga caataaccct gataaatgct 4380
tcaataatag cacgtgctaa aacttcattt ttaatttaaa aggatctagg tgaagatcct 4440
ttttgataat ctcatgacca aaatccctta acgtgagttt tcgttccact gagcgtcaga 4500
ccccgtagaa aagatcaaag gatcttcttg agatcctttt tttctgcgcg taatctgctg 4560
cttgcaaaca aaaaaaccac cgctaccagc ggtggtttgt ttgccggatc aagagctacc 4620
aactcttttt ccgaaggtaa ctggcttcag cagagcgcag ataccaaata ctgttcttct 4680
agtgtagccg tagttaggcc accacttcaa gaactctgta gcaccgccta catacctcgc 4740
tctgctaatc ctgttaccag tggctgctgc cagtggcgat aagtcgtgtc ttaccgggtt 4800
ggactcaaga cgatagttac cggataaggc gcagcggtcg ggctgaacgg ggggttcgtg 4860
cacacagccc agcttggagc gaacgaccta caccgaactg agatacctac agcgtgagct 4920
atgagaaagc gccacgcttc ccgaagggag aaaggcggac aggtatccgg taagcggcag 4980
ggtcggaaca ggagagcgca cgagggagct tccaggggga aacgcctggt atctttatag 5040
tcctgtcggg tttcgccacc tctgacttga gcgtcgattt ttgtgatgct cgtcaggggg 5100

gcggagccta tggaaaaacg ccagcaacgc ggccttttta cggttcctgg ccttttgctg 5160
gccttttgct cacatgttct t 5181

## Description

### TECHNICAL FIELD

The present invention relates to a medicament and a method for amelioration of a cerebral function. More particularly, the present invention relates to a medicament for amelioration of a cerebral function which contains a cellular growth factor or the like as an active ingredient, and a method for amelioration of a cerebral function.

### BACKGROUND ART

The brain is the most important organ among various organs, but it is also the least studied field. Within this field, control of cerebral functions has not been fully explained in relation to its actual mechanisms. In particular, it is unknown if amelioration of physiological conditions of the brain (for example, neoangiogenesis) will directly result in amelioration of cerebral functions in any ways, and there are a number of unknown points with regard to the control of cerebral functions (for example, amelioration, prevention of deterioration and the like).

Cerebral occlusive diseases, occlusive disease of the circle of Willis and the like caused by atherosclerosis in the artery of the brain often cause chronic reduction in cerebral blood flow. This state may not only cause subsequent cerebral ischemia but also neuropathological alteration including dementia, that is, decline in cerebral functions (Sekhon LH, Morgan MK, Spence I, Weber NC., Stroke 25, 1022-1027 (1994); Stroke 29, 1058-1062 (1998); Stroke 24, 259-264 (1993) ; and Kalaria RN, Bhatti SU, Lust WD, Perry G., Ann. N.Y, Acad. Sci. 695, 190-193 (1993). However, no effective therapy for the amelioration of such decline in cerebral functions has been established.

Development of a new blood vessel or neoangiogenesis is initiated with the activation of endothelial cells in a parent blood vessel. A growth factor which has been shown to stimulate such neoangiogenesis *in vivo* and possess mitogenic effect on endothelial cells in vitro is referred to as "angiogenic growth factor". Involvement of angiogenic growth factors in therapy was published for the first time by Folksman et al. in an article (Folksman et al., N. Engl. J. Med. 285, 1182-1186 (1971)). Further, through subsequent studies, it has been confirmed that the development of collateral vessels may be enhanced and/or promoted in animal models with myocardial ischemia or limb ischemia, using a recombinant angiogenic factor, for example, fibroblast growth factor (FGF) family (Science257, 1401-1403 (1992) and Nature 362, 844-846 (1993)), endothelial cell growth factor (J. Surg. Res, 54, 575-583 (1993)), vascular endothelial growth factor (VEGF) (Circulation 90, II-228-II-234 (1994)) and the like. Furthermore, the present inventors found that HGF also acts as an endothelium-specific growth factor , which is similar to VEGF (J. Hypertens. 14, 1067-1072 (1996)).

Strategy of using an angiogenic growth factor as described above for therapy of vascular disorder is referred to as "therapeutic angiogenesis". More recently, this strategy has been applied to human ischemic diseases. However, the effectiveness of this strategy for amelioration of the decline in cerebral functions or for the prevention of deterioration thereof is unknown at the present time and is unpredictable from the knowledge obtained so far.

A hepatocyte growth factor (HGF) is a pleiotrophic cytokine which exhibits mitogenic activity, motility enhancing activity and morphogenetic activity on various cells (Nature 342, 440-443 (1989)).

Regarding effects of HGF on brain, the following reports have been made. That is, it is known that HGF and transmembrane tyrosine kinase c-Met/HGF receptor are expressed in various regions in a brain, functional binding between HGF and c-Met enhances survival of neurons in a primarily cultured hippocampus, neurite outgrowth is induced in the development of neurons in vitro (J. Cell. Biol. 126, 485-494 (1994) and Japanese Laid-Open Publication No. 7-89869). Recently, it has been reported that HGF is induced in ischemic neurons (Brain Res. 799, 311-316 (1998)), a recombinant HGF has neuroprotective effects against tardive neuronal death after ischemia in the hippocampus, and that continuous injection of recombinant HGF into a brain is effective for reducing the degree of an infarction (J. Cereb. Blood Flow Metab. 18, 345-348 (1998). In view of these knowledge, it is believed that an HGF can act as an important neurotrophic factor during cerebral ischemia. However, the effectiveness of HGF for the amelioration of cerebral functions is unknown and is unpredictable.

On the other hand, a vascular endothelial growth factor (VEGF) is a dimeric glycoprotein which has a mitogenic effect on an endothelial cell, and also has an ability of enhancing vascular permeability. VEGF has direct and specific mitogenic effects on an endothelial cell (Biochem. Biophys. Res. Commun., 161, 851-858 (1989)). Binding sites of a VEGF including tyrosine kinase receptor, Flt, Flk-1 and KDR are found on endothelial cells, but not on other types of cells, and thus the effects of a VEGF are limited to endothelial cells.

Regarding the effects of VEGF on brain, it has been reported that VEGF is rapidly induced in a brain due to ischemic disorder in the central nervous system (Mol. Cell. Biol. 16, 4604-4613 (1996)), and that administration of a recombinant VEGF to a brain surface was effective for reducing the degree of infarction (J. Cereb. Blood Flow Metab. 18, 887-895 (1998)). However, the exact mechanism has not been clarified, and hence the effectiveness of HGF for the amelioration of cerebral functions is also unknown and unpredictable.

Most effects of FGF on brain are also unknown. Effectiveness of FGF for the amelioration of cerebral functions is unknown and unpredictable.

From another viewpoint, while the above HGF, VEGF, FGF and the like act as cellular growth factors, they are also strong angiogenic growth factors, as described above (J. Cell. Biol. 119, 629-641 (1992)). Accordingly, neoangiogenesis is believed to play an important role in the recovery from cerebral ischemia or in the prevention of future paroxysm. However, it is unknown and unpredictable if neoangiogenesis is related to amelioration of decline in cerebral functions or prevention of deterioration of cerebral functions (for example, amelioration of memory function and amelioration of learning function).

Further, since recombinant angiogenic growth factors are rapidly used up, they must be continuously injected into the brain. This operation is very dangerous and difficult under clinical circumstances. It is thus considered that continuous expression and secretion of angiogenic growth factor in an ischemic brain or therearound using transgenesis would be rational, if possible. However, there has been no cases of application of HGF or VEGF to cerebral ischemic disorders (gene therapy), and almost no study on cerebral functions, which reflects specificity of tissue of a brain, has been performed so far. It is therefore unknown whether HGF and VEGF are effective for the amelioration of cerebral functions in reality.

In the present time, when solutions have been found for almost all diseases, cerebral diseases and disorders may be considered as the last area in which only a few solutions have been found. In particular, the demand for therapy and prophylaxis of cerebral diseases and disorders for the purpose of amelioration of decline in cerebral functions and prevention of deterioration of cerebral functions have increased year after year. Among cerebral diseases,those caused by atherosclerosis in cerebral arteries, occlusive disease of the circle of Willis and the like often cause chronic cerebral hypoperfusion. This not only leads to cerebral ischemic events but also results in neuropathological alterations, including dementia (Sekhon LH, Morgan MK, Spence I, Weber NC., Stroke 25, 1022-1027 (1994); Stroke 29, 1058-1062 (1998); Stroke 24, 259-264 (1993); and Kalaria RN, Bhatti SU, Lust WD, Perry G., Ann. N.Y, Acad. Sci. 695, 190-193 (1993). However, no effective treatment for the amelioration of cerebral functions has been established.

Thus, although it has been reported that an angiogenic growth factor such as vascular endothelial growth factor (VEGF), fibroblast growth factor (FGF) and hepatocyte growth factor (HGF) stimulates the development of a collateral vessel in animal models with cerebral ischemia through preclinical studies (Harrigan MR, Ennis SR, Masada T, KeepRF., Neurosurgery. 2002;50:589-98; LyonsMK, Anderson RE, Meyer FB., Brain Res. 1991;558:315-20; and Yoshimura S, et al., Hypertension. 2002;39:1028-34.), it is unknown and unpredictable if such angiogenic growth factors are effective for the amelioration of cerebral functions in reality.
Non-Patent Document 1 : Sekhon LH, Morgan MK, Spence I, Weber NC., Stroke 25, 1022-1027 (1994)
Non-Patent Document 2: Kurumatani T, Kudo T, Ikura Y, Takeda M., Stroke 29, 1058-1062 (1998)
Non-Patent Document 3: Kudo T, Takeda M, Tanimukai S, Nishimura T., Stroke 24, 259-264 (1993)
Non-Patent Document 4: Kalaria RN, Bhatti SU, Lust WD, PerryG., Ann. N.Y, Acad. Sci. 695, 190-193 (1993)
Non-Patent Document 5: Folksman et al., N. Engl. J. Med. 285, 1182-1186 (1971)
Non-Patent Document 6: Yanagisawa-Miwa A, Uchida Y, et al., Science 257, 1401-1403 (1992)
Non-Patent Document 7: Nabel EG, et al., Nature 362, 844-846 (1993)
Non-Patent Document 8: Pu LQ, Sniderman AD, Arekat Z, Graham AM, Brassard R, Symes JF., J. Surg. Res, 54, 575-583 (1993)
Non-Patent Document 9: Takeshita S, Pu LQ, Stein LA, Sniderman AD, Bunting S, Ferrara N, Isner JM, Symes JF, Circulation 90, II-228-II234 (1994)
Non-Patent Document 10: Y. Nakamura, R. Morishita, J. Higaki, I. Kida, M. Aoki, A. Moriguchi, K. Yamada, S. Hayashi, Y. Yo, H. Nakao, K. Matsumoto, T. Nakamura and T. Ogihhara, J. Hypertens. 14, 1067-1072 (1996)
Non-Patent Document 11: T. Nakamura, T. Nishizawa, M. Hagiya, T. Seki, M. Shimonishi, A. Sugimura, K. Tashiro and S. Shimizu, Nature 342, 440-443 (1989)
Non-Patent Document 12: Jung W, Castren E, Odenthal M, Vande Woude GF, Ishii T, Dicenes HP, Lindholm D, Schirmacher P. J, Cell. Biol. 126, 485-494 (1994)
Non-Patent Document 13: Hayashi T, Abe K, Sakurai M, Itoyama Y., Brain Res. 799, 311-316 (1998)
Non-Patent Document 14: Morita F, Et al., J. Cereb. Blood Flow Metab. 18, 345-348 (1998)
Non-Patent Document 15: Ferrara, N. and Henzel, WJ, Biochem. Biophys. Res. Commun., 161, 851-858 (1989)
Non-Patent Document 16: Forsythe, JA, Jiang, BH, Iyer, NV, Agani, F, Leung, SW, Koos, RD, and Semenza, GL, Mol. Cell. Biol., 16, 4604-4613 (1996)
Non-Patent Document 17: Hayashi, T., Abe, K., & Itoyama, Y. J. Cereb. Blood Flow Metab. 18, 887-895 (1998)
Non-Patent Document 18: Bussolino, F., et al., J. Cell. Biol. 119, 629-641 (1992)
Non-Patent Document 19: Harrigan MR, Ennis SR, Masada T, Keep RF., Neurosurgery. 2002;50:589-98
Non-Patent Document 20: Lyons MK, Anderson RE, Meyer FB., Brain Res. 1991;558:315-20.
Non-Patent Document 21: Yoshimura S, et al., Hypertension. 2002;39:1028-34.
Patent Document 1: Japanese Laid-Open Patent Application No. 7-89869

### DISCLOSURE OF THE INVENTION

### PROBLEMS TO BE SOLVED BY THE INVENTION

The problemof the present invention is to provide a novel therapeutic agent for the amelioration of cerebral functions or the prevention of deterioration of cerebral functions and a novel administration method of such a therapeutic agent.

### MEANS FOR SOLVING THE PROBLEMS

The present invention has solved the above problem by providing a composition containing a growth factor such as HGF (hepatocyte growth factor) gene and/or VEGF (vascular endothelial growth factor) gene as an active ingredient, which has acts as therapy for disorders of cerebral functions or prevention of deterioration of cerebral functions, and enhances neurite outgrowth or synaptogenesis.

Therefore, the present invention relates to the following items:
(1) A composition for prevention of deterioration of a cerebral function or amelioration of a cerebral function, the composition including a cellular growth factor as an active ingredient.
(2) Thecompositionaccordingtoitem 1, wherein the cellular growth factor has action of inducing vascular growth.
(3) Thecompositionaccordingtoitem 1, wherein the cellular growth factor is selected from the group consisting of: vascular endothelial growth factor (VEGF); fibroblast growth factor (FGF); and hepatocyte growth factor (HGF).
(4) Thecompositionaccordingtoitem 1, wherein the cellular growth factor is a hepatocyte growth factor (HGF).
(5) Thecompositionaccordingtoitem 1, wherein the cerebral function is a cognitive function or a motor function.
(6) Thecompositionaccordingtoitem 1, wherein the cerebral function is affected by cognitive dysfunction or motor dysfunction due to cerebral infarction, cerebral blood flow disorder, cerebral hemorrhage or cerebrovascular disorder.
(7) Thecompositionaccordingtoitem 1, wherein the cerebral function is selected from the group consisting of a memory function and a spatial learning function.
(8) The composition according to item 1, wherein prevention of deterioration of the cerebral function or amelioration of the cerebral function is achieved by activation of astrocytes.
(9) Thecompositionaccordingtoitem 1, wherein the cellular growth factor is provided in the form of a protein or in the form of a nucleic acid.
(10) The composition according to item 1, wherein the cellular growth factor is administered with a viral envelope.
(11) The composition according to item 10, wherein the viral envelope is inactivated.
(12) The composition according to item 10, wherein the viral envelope is an envelope of RNA virus.
(13) The composition according to item 10, wherein the viral envelope is an envelope of *Paramyxoviridae* virus.
(14) The composition according to item 10, wherein the viral envelope is an envelope of HVJ.
(15) The composition according to item 10, wherein the cellular growth factor is contained in the viral envelope.
(16) The composition according to item 1, which is delivered by administration to subarachnoid space or by intracisternal administration.
(17) The composition according to item 1, which is administered six days after development of cerebral infarction, cerebral blood flow disorder, cerebral hemorrhage, cerebrovascular disorder or disorder of a cerebral function.
(18) The composition according to item 1, which is administered six days after development of cerebral infarction, cerebral blood flow disorder, cerebral hemorrhage, cerebrovascular disorder or disorder of a cerebral function, wherein the cellular growth factor is in the form of a nucleic acid.
(19) A method for prevention of deterioration of a cerebral function or amelioration of a cerebral function, the method including the step of:
   (A) administering a cellular growth factor to a patient.
(20) The method according to item 19, wherein the cellular growth factor has action of inducing vascular growth.
(21) The method according to item 19, wherein the cellular growth factor is selected from the group consisting of: vascular endothelial growth factor (VEGF); fibroblast growth factor (FGF); and hepatocyte growth factor (HGF).
(22) The method according to item 19, wherein the cellular growth factor is a hepatocyte growth factor (HGF).
(23) The method according to item 19, wherein the cellular growth factor is administered with a viral envelope.
(24) The method according to item 23, wherein the viral envelope is an envelope of HVJ.
(25) The method according to item 19, wherein the cerebral function is a cognitive function or a motor function.
(26) The method according to item 19, wherein the cerebral function is affected by cognitive dysfunction or motor dysfunction due to cerebral infarction, cerebral blood flow disorder, cerebral hemorrhage or cerebrovascular disorder.
(27) The method according to item 19, wherein the cerebral function is selected from the group consisting of a memory function and a spatial learning function.
(28) The method according to item 19, wherein the cellular growth factor is in the form of a protein or in the form of a nucleic acid.
(29) The method according to item 19, wherein the cellular growth factor is delivered by administration to subarachnoid space or by intracisternal administration.
(30) The method according to item 19, wherein the cellular growth factor is administered six days after development of cerebral infarction, cerebral blood flow disorder, cerebral hemorrhage, cerebrovascular disorder or disorder of a cerebral function.
(31) A use of a cellular growth factor in the production of a,medicament for prevention of deterioration of a cerebral function or amelioration of a cerebral function.
(32) The use according to item 31, wherein the cellular growth factor is selected from the group consisting of: vascular endothelial growth factor (VEGF); fibroblast growth factor (FGF); and hepatocyte growth factor (HGF).
(33) The use according to item 31, wherein the cellular growth factor is a hepatocyte growth factor (HGF).
(34) The use according to item 31, wherein the cellular growth factor is in the form of a protein or in the form of a nucleic acid.
(35) A composition comprising a cellular growth factor used for enhancement of neurite outgrowth or synaptogenesis..
(36) The composition according to item 35, wherein the cellular growth factor is selected from the group consisting of: vascular endothelial growth factor (VEGF); fibroblast growth factor (FGF); and hepatocyte growth factor (HGF).
(37) The composition according to item 35, wherein the cellular growth factor is a hepatocyte growth factor (HGF).
(38) The composition according to item 35, wherein the cellular growth factor is in the form of a protein or in the form of a nucleic acid.
(39) A method for enhancement of neurite outgrowth or synaptogenesis, the method including the step of:
   (A) administering a cellular growth factor to an individual for which enhancement of neurite outgrowth or synaptogenesis is necessary.
(40) The method according to item 39, wherein the cellular growth factor is selected from the group consisting of: vascular endothelial growth factor (VEGF); fibroblast growth factor (FGF); and hepatocyte growth factor (HGF).
(41) The method according to item 39, wherein the cellular growth factor is a hepatocyte growth factor (HGF).
(42) The method according to item 39, wherein the cellular growth factor is in the form of a protein or in the form of a nucleic acid.
(43) A use of a cellular growth factor in the production of a medicament for enhancement of neurite outgrowth or synaptogenesis.
(44) The use according to item 43, wherein the cellular growth factor is selected from the group consisting of: vascular endothelial growth factor (VEGF); fibroblast growth factor (FGF); and hepatocyte growth factor (HGF).
(45) The use according to item 43, wherein the cellular growth factor is a hepatocyte growth factor (HGF).
(46) The use according to item 43, wherein the cellular growth factor is in the form of a protein or in the form of a nucleic acid.

The present inventors performed in vivo studies testing whether cerebral functions can be ameliorated by administration of a cellular growth factor such as HGF, FGF and VEGF. As a result, the present inventors clarified that administration of a cellular growth factor resulted in significant amelioration of cerebral functions such as memory function and spatial learning function, and enhanced neurite outgrowth or synaptogenesis, in particular with regard to the region of cerebral infarction and therearound. The present invention was completed based on the above knowledge.

Hereinafter, preferred embodiments of the present invention will be described. It should be recognized that those skilled in the art can appropriately carry out the embodiments or the like based on the explanation of the present invention and well-known ordinary techniques in the field of the art and can also readily understand actions and effects attained by the present invention.

Therefore, it is understood that these and other advantages of the present invention will be apparent to those skilled in the art by reading and understanding the following detailed description with reference to the attached drawings and the like.

### EFFECTS OF THE INVENTION

The present invention provides a composition for the amelioration of decline in cerebral functions or prevention of deterioration of cerebral functions and enhancement of neurite outgrowth or synaptogenesis, in particular with regard to the region of cerebral infarction or therearound. No such composition which is directly related to the control of cerebral functions or compositions for the enhancement of neurite outgrowth or synaptogenesis, in particular with regard to the region of cerebral infarction or therearound, has been known so far. It thus indicates that the present invention has significant effects which have been conventionally impossible.

### BRIEF DESCRIPTION OF THE DRAWINGS

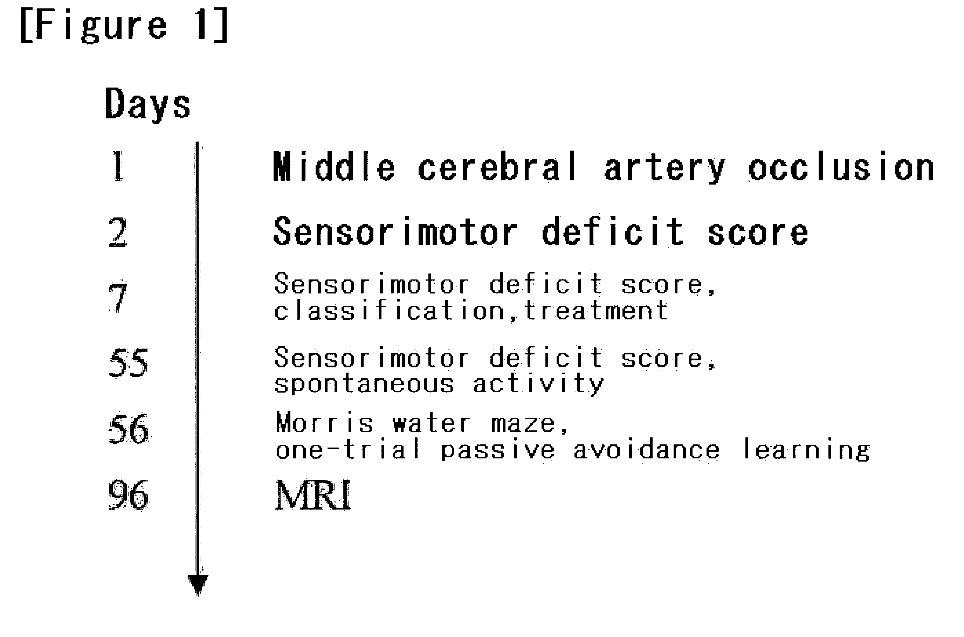

Figure **1** shows a protocol of treatment. In order to test therapeutic effects of hepatocyte growth factor (HGF) gene on cognitive dysfunction, HGF was injected seven days after the paroxysm. On day 56 after ischemia, cognitive dysfunction was evaluated by Morris water maze and one-trial passive avoidance learning.

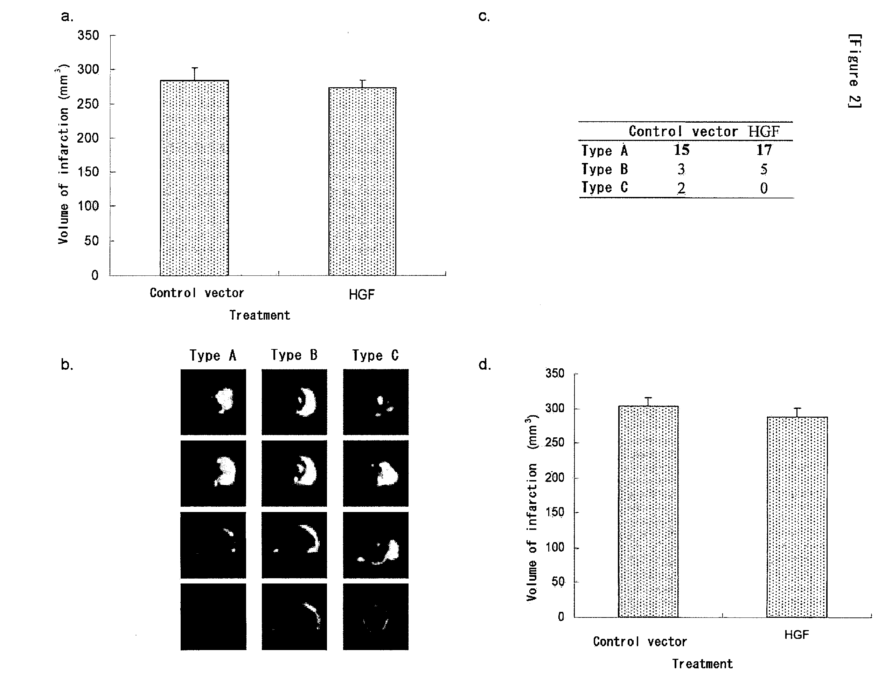

Figure **2** shows nuclear magnetic resonance image of a brain. **(a)** shows the degree of infarction of a rat by T2 weightedimage. There was no difference between both groups. A region with high intensity indicates the region of infarction. **(b)** shows representative images of T2 weighted image of the frontal region of rat brain. These images are divided into three types. Type A: a region with high intensity is found in the cortex and basal ganglia. Type B: a region with high intensity is found in the cortex or some portions of the basal ganglia. Type C: a region with high intensity is found in the cortex and some portions of the basal ganglia. **(c)** shows the number of rats for each type. It indicates that most rats were type A, that is, almost none of the rats were of type B or C. Behavior of type A was analyzed. **(d)** shows the degree of infarction in type A. No significant difference was found.

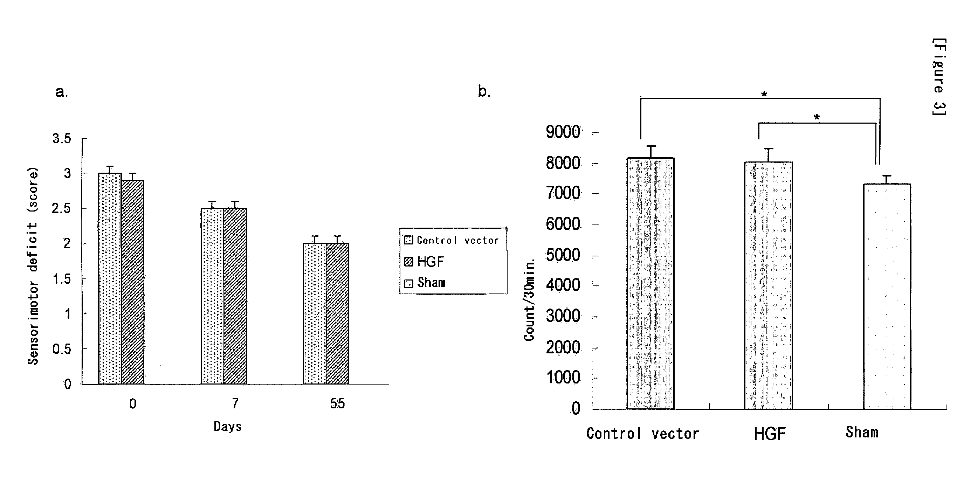

Figure 3 shows sensorimotor deficit and spontaneous activity. **(a)** shows a score of sensorimotor deficit. Sensorimotor deficit was spontaneously recovered from each group, and there was no significant differences. **(b)** shows spontaneous activity. For rats subjected to medium cerebral artery occlusion, the number of crossover points increased in the open field, but there was no significant differences between other groups (for vehicle-treated rats, n=15, for HGF-treated rats, n=17 and for sham-treated rats, n=10).

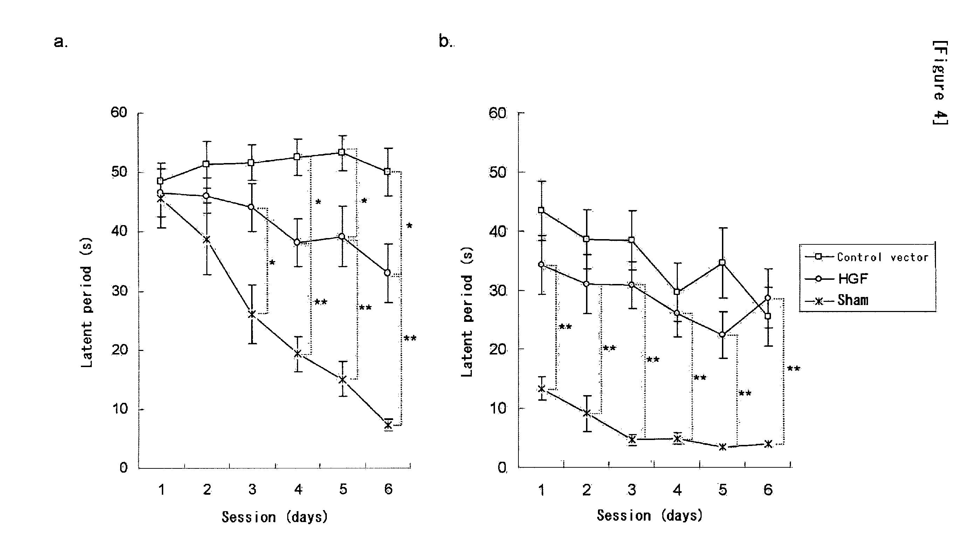

Figure **4** shows Morris water maze test. **(a)** shows the invisible platform test. In comparison to the sham-treated rats, almost none of the rats subj ected to medium cerebral artery occlusion reached the invisible platform. On day 4-6, it was significant that the HGF-treated rats could reach faster than the vehicle-treated rats. **(b)** shows the visible platform test. Unlike the invisible platform test, rats from both groups could reach the platform. There was no significant difference between these groups (for vehicle-treated rats, n=15, for HGF-treated rats, n=17, and for sham-treated rats, n=10, *p<0.05).

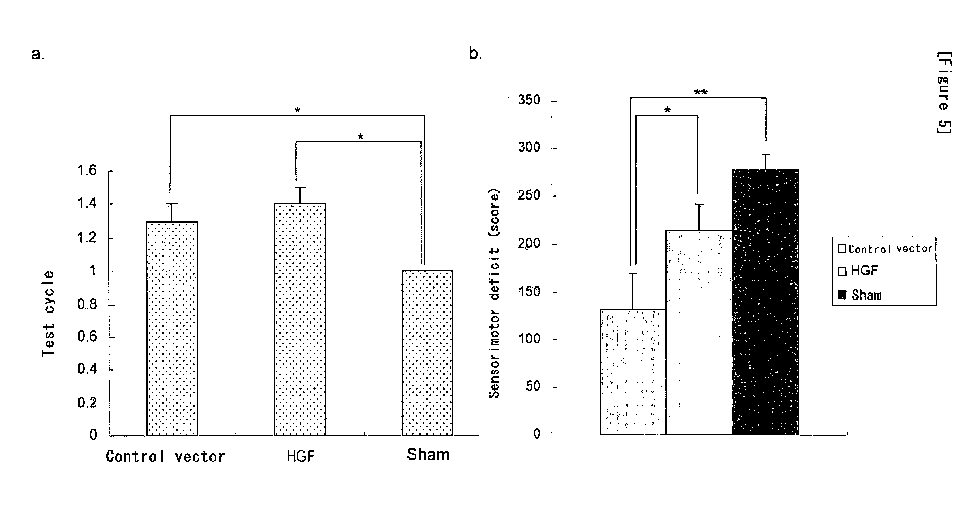

Figure **5** shows one-trial passive avoidance learning. **(a)** shows the acquisition trial. The number of learning sessions required for the acquisition of memory was significantly greater for the rats subjected to medium cerebral artery occlusion. However, no significant differences was observed between vehicle-treated rats and HGF-treated rats. **(b)** shows the retention trial. Latent period during which the rats stayed in the light chamber was longer for HGF-treated rats than vehicle-treated rats on day 1 and day 3 (for vehicle-treated rats, n=15, for HGF-treated rats, n=17, and for sham-treated rats, n=10, *p<0.05).

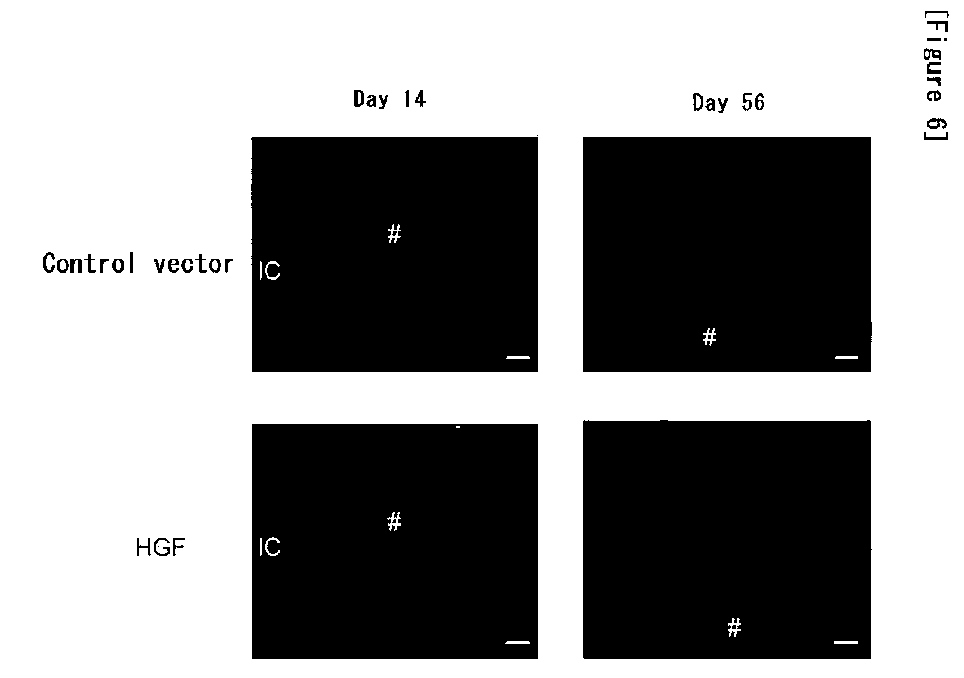

Figure **6** shows the immunohistochemistry of GFAP. It shows representative immunohistochemical images of GFAP in the ipsilateral cortex on day 14 and 56. In a peri-infarcted region of HGF-treated rats (#), immunoreactivity was increased on day 14, and decreased on day 56. IC is the ischemic center (for each group, n=3 and bar=100µm).

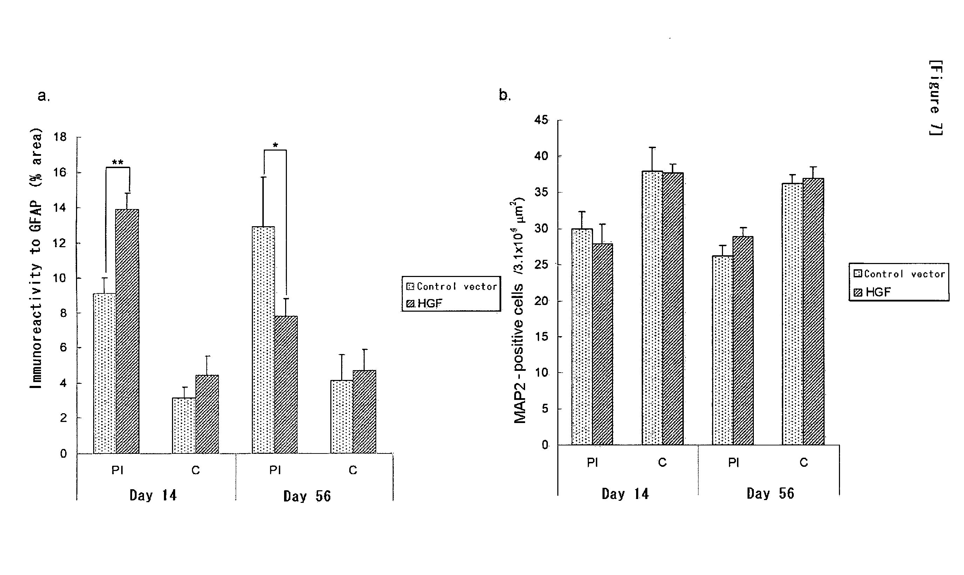

Figure **7** shows quantitative analysis of MAP-2 positive cell and GFAP-positive cell. It shows quantification of MAP-2 positive cell and GFAP-positive cell in the cortex. While there was no significant difference in the number of MAP2-positive cells between the two groups, immunoreactivity of HGF-treated rats in response to GFAP was higher than vehicle-treated rats on day 14, but lower than vehicle-treated rats on day 56 (PI: peri-infarcted region; C: contralateral region, n=3 for each group. *p<0.05, **p<0.01).

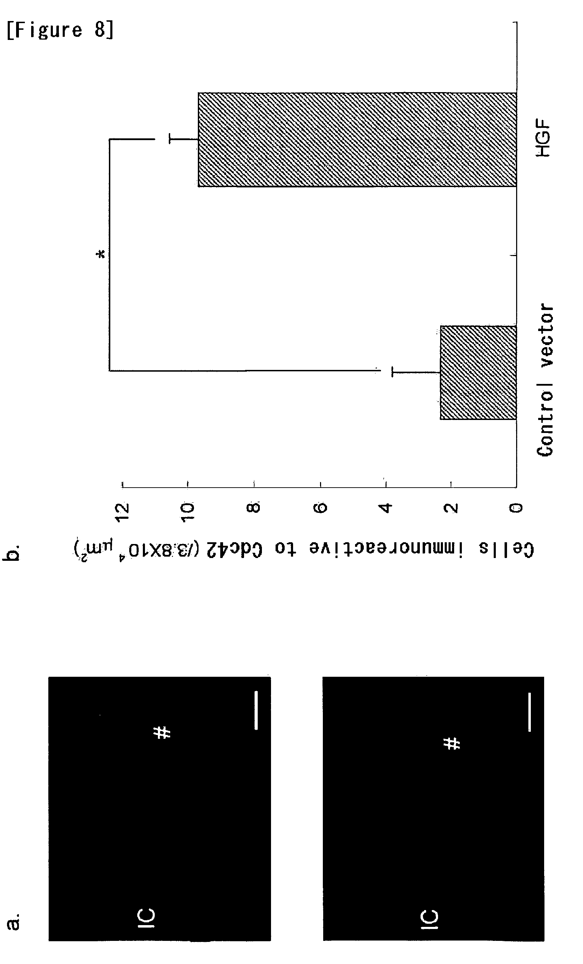

Figure **8** shows the immunohistochemistry of Cdc42.
   **(a)** shows representative immunohistochemical images of Cdc42 in the ipsilateral cortex on day 14. In a peri-infarcted region of HGF-treated rats (#), immunoreactivity with Cdc42 significantly increased (IC: ischemic center, Bar=100µm).
   **(b)** shows the quantitative analysis of Cdc42-immunoreactive cell. The number was counted in the peri-infarcted region (#). For HGF-treated rats, the number of Cdc42-immunoreactive cells increases (n=3 for each group, *p<0.05).

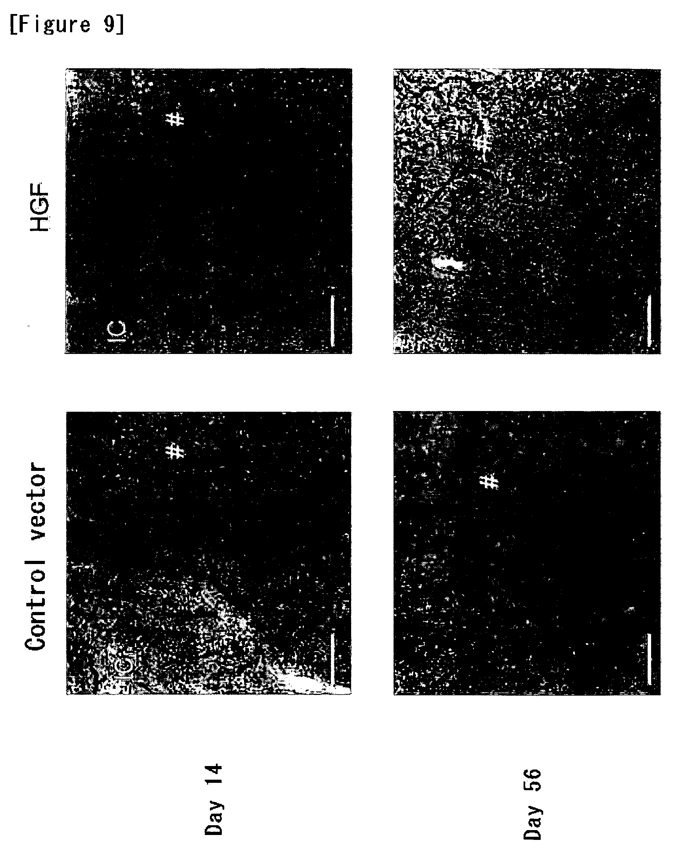

Figure **9** shows representative images of ALP staining. Coronal sections stained with ALP in the ipsilateral cortex are shown. The structure of arteries in the peri-infarcted region (#) was not changed as of day 14. However, the arteries of HGF-treated rats showed more complicated patterns on day 56 (IC: ischemic center).

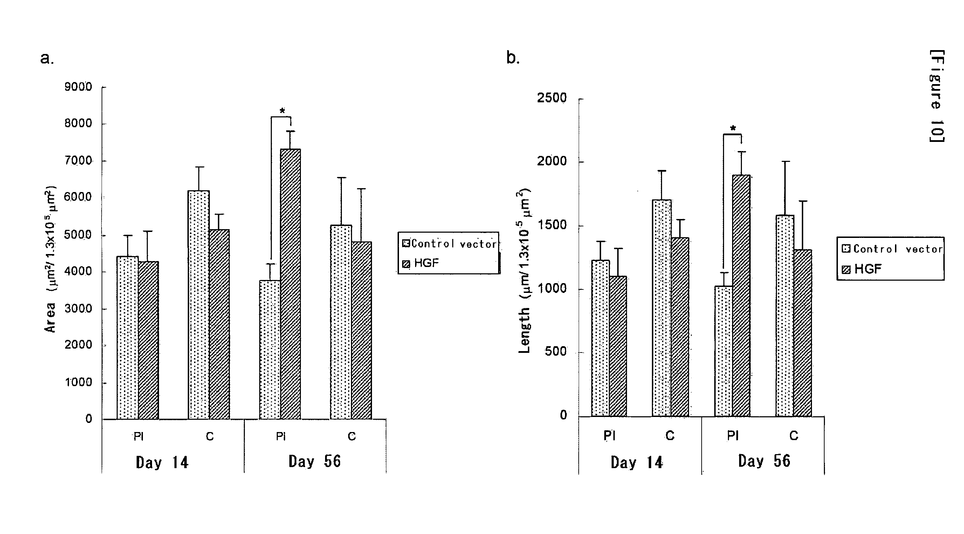

Figure **10** shows quantitative analysis of ALP staining. Quantification of a blood vessel in the cortex stained with ALP is shown. The area and the length of the blood vessels were different on day 14, and those of HGF-treated rats increased significantly on day 56 (n=3, *p<0.05).

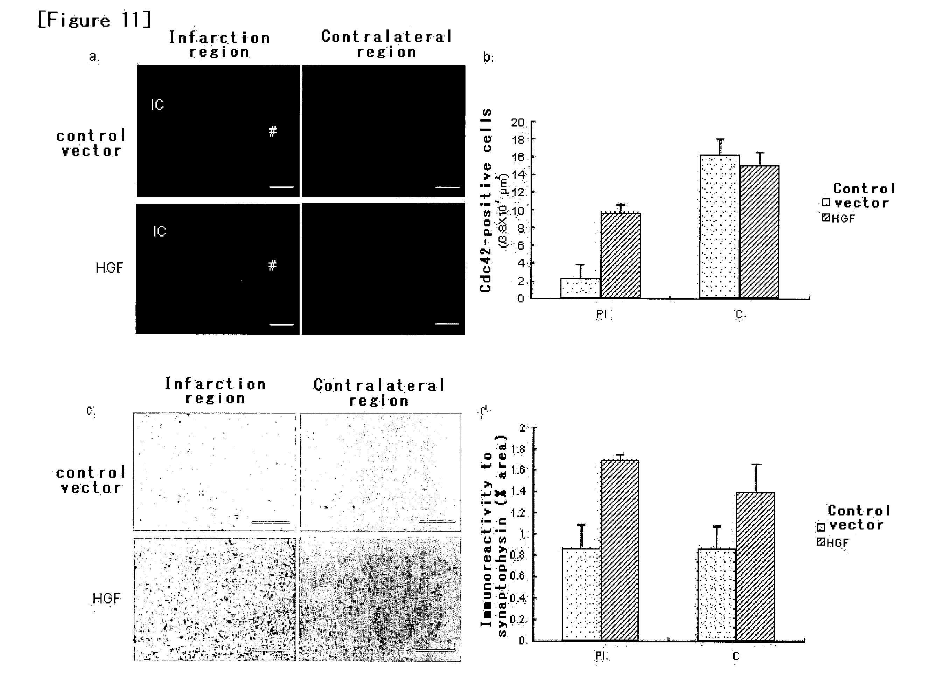

Figure **11** shows representative immunohistochemical images of Cdc42 and synaptophysin in the infarction region and contralateral region when the control gene or HGF gene is administered. **(a)** shows results of immunostaining the region for Cdc42. The left images of **(a)** show the infarction region and the right images of **(a)** show the contralateral region. The upper images of **(a)** show results in the case of administration of a control gene (vector), and the lower images of **(a)** show results in the case of administration of a HGF gene. IC is the ischemic center. # shows the peri-infarcted region, and the bar indicates 100µm. **(b)** shows the number of Cdc42-positive cells in the infarction region (Pi) and the contralateral region (C). * indicates statistical significance (p<0.05). The cross-hatched portion indicates the HGF gene-administered group and the dotted portion indicates the control vector-administered group. **(c)** shows the results of immunostaining the region for synaptophysin. The left images of **(c)** show the infarction region and the right images of **(c)** show the contralateral region. The upper images of **(c)** show results of administrating a control gene (vector), and the lower images of **(c)** show results of administrating a HGF gene. The bar indicates 100µm. **(d)** shows the number of synaptophysin-positive cells in the infarction region (Pi) and the contralateral region (C). * indicates statistical significance (p<0.05). The cross-hatched portion indicates the HGF-administered group, and the dotted portion indicates the control vector-administered group.

(Sequence Listing)
SEQ ID NO:1: nucleic acid sequence of HGF
SEQ ID NO:2: amino acid sequence of HGF
SEQ ID NO:3: nucleic acid sequence of VEGF
SEQ ID NO:4: amino acid sequence of VEGF
SEQ ID NO:5 nucleic acid of aFGF
SEQ ID NO:6: amino acid sequence of aFGF
SEQ ID NO:7: pcDNA3.1(-)HGF
SEQ ID NO:8: pVAX1HGF/MGB1

### BEST MODE FOR CARRYING OUT THE INVENTION

Embodiments described below are provided for better understanding of the present invention, and it should be understood that the scope of the present invention should not be limited to the following description. Therefore, it is apparent that those skilled in the art can appropriately make modification within the scope of the present invention considering the description provided herein.

Hereinafter, the present invention will be described with reference to the attached drawings and by way of examples, if necessary. It should be understood that, throughout herein, an expression in a singular form also includes a concept thereof in a plural form, unless particularly mentioned otherwise. Therefore, it should be understood that an expression in a singular form (for example, "a", "an", "the" and the like in the English language) includes a concept thereof in a plural form, unless particularly mentioned otherwise. It should also be understood that terms used herein are used in a sense typically used in the field of the art, unless particularly mentioned otherwise. Therefore, unless otherwise defined, all of the technical terms and scientific terms used herein have the same meaning as typically understood by those skilled the art in the field to which the present invention belongs. If the meanings are contradictory, the present specification (including definitions) is prior.

### (Terms)

Hereinafter, definitions of the terms particularly used herein will be described.

As used herein, "cerebral function" refers to a complicated activity performed by many parts of a cerebrum. Examples of such a function include language function, cognitive function, motor function, memory function, learning function, attention function and the like.

As used herein, "cognitive function" refers to ability of an organism to recognize identity of other organisms. "Cognitive function" is confirmed by, for example, behavioral tests, spontaneous activity tests, Morris water maze tests and the like.

As used herein, "motor function" refers to management of motion by the nerve or brain. "Motor function" can be confirmed by, for example, behavioral tests, spontaneous activity tests, Morris water maze tests and the like.

As used herein, "memory function" refers to a function of the brain or nervous system to memorize a previously experienced state or information. "Memory function" can be confirmed by, for example, behavioral tests, Morris water maze tests, passive avoidance learning and the like.

As used herein, "learning function" refers to a function of the brain or nervous system to memorize and use a previously experienced state. "Learning function" can be confirmed by, for example, behavioral tests, Morris water maze tests, passive avoidance learning and the like.

"Hepatocyte growth factor" (HGF) used in the present invention is described in, for example, Nature, 342, 440 (1989), Japanese Patent Publication No. 2577091, Biochem. Biophys. Res. Commun., 163, 967 (1989), Biochem. Biophys. Res, Commun., 172, 321 (1990) and the like. Representative sequences will be set forth in SEQ ID NOS:1 and 2 (nucleic acid sequence and amino acid sequence respectively). To express such an HGF, HGF cDNA can be incorporated into an appropriate expression vector, as will be described below (nonviral vector and viral vector). Base sequences of cDNA encoding an HGF here are also registered in a database such as Genbank, in addition to those described in the literatures described above. Accordingly, based on these sequence information, for example, RT-PCR reaction can be performed with respect to a mRNA derived from a liver or leucocyte using an appropriate DNA portion as a PCR primer, thereby cloning a cDNA of an HGF. Those skilled in the art would be able to readily perform such cloning in accordance with a basic reference, for example, Molecular Cloning 2nd Edt. , Cold Spring Harbor LaboratoryPress (1989) and the like.

Further, an HGF of the present invention is not limited to the above HGF. Any protein may be used as an HGF of the present invention, as long as the expressed protein is a protein having substantially the same action as an HGF. For example, an HGF of the present invention can also encompass a protein having substantially the same action as an HGF and consists of an amino acid sequence including one or a plurality of (preferably, several) amino acid substitution, deletion and/or addition with respect to an amino acid sequence of a well-known HGF protein such as a protein encoded by the above cDNA.

Further, a nucleic acid (DNA and RNA) encoding these proteins can be used instead of the above protein in the present invention. As used herein, "form of protein" with regard to a cellular growth factor refers to a cellular growth factor itself (that is, a protein) or a protein having substantially the same action as the cellular growth factor. "Form of nucleic acid" includes a nucleic acid encoding a cellular growth factor itself or a protein having substantially the same action as the cellular growth factor, or a nucleic acid which hybridizes with such a nucleic acid under stringent conditions (herein, these nucleic acids may be represented as a gene), and indicates a form such that, when administered to a subject, a protein encoded by such a nucleic acid is expressed in the subject. In the case of administration to a subject in a "form of nucleic acid", a protein encoded by the nucleic acid is expressed in the subject, and the protein attains actions as a cellular growth factor.

"Vascular endothelial growth factor (VEGF) " as used herein is described in, for example, Science, 219, 983 (1983), J. Clin. Invest., 84, 1470 (1989), Biochem. Biophys. Res. Commun., 161, 851 (1989) and the like. Representative sequences will be set forth in SEQ ID NOS:3 and 4 (a nucleic acid sequence and amino acid sequence respectively). A VEGF can be prepared using, for example, a cDNA of VEGF incorporated into an appropriate expression vector, as will be described below (nonviral vector and viral vector). Regarding human VEGF, the existence of four subtypes (VEGF121, VEGF165, VEGF189 and VEGF206) have been reported through alternative splicing in transcription (Science, 219, 983 (1983), J. Clin. Invest., 84, 1470 (1989) and Biochem. Biophys. Res. Commun., 161, 851 (1989)). Although any of these VEGFs can be used in the present invention, VEGF165 gene is preferred in view of its most intense biological activity. Further, similar to the HGF described above, even genes obtained by modification or the like with respect to the genes of these VEGFs are encompassed in the category of VEGF of the present invention, as long as the genes encode a protein having the actions of VEGF.

Those skilled in the art would be able to readily clone VEGF as well as HGF based on the sequence described in a literature (for example, Science, 246, 1306 (1989)) and sequence information registered in databases, and would also be able to readily make modification thereof and the like.

"Fibroblast growth factor (FGF) " as used herein is described in, for example, Diilber MS, et al. (1994) Exp Hematol 22(12):1129-33, and is expressed in various normal cells and cancer cells. As gene products thereof, at least nine associated growth factors, bFGF (basic), aFGF (acidic), FGF3, which is an int-2 gene product, FGF4 , which is a K-fgf/hst1 gene product, FGF5, FGF6, which are hst2 gene products, keratinocyte growth factor (KGF), androgen-induced growth factor (AIGF) and FGF9, which have similar structure and functions, such as strong binding to heparin, are known. Representative sequences will be set forth in SEQ ID NOS:5 and 6 (a nucleic acid sequence and amino acid sequence=aFGF respectively).

As used herein, "neoangiogenesis" refers to the formation of a new blood vessel and an activity of such formation.

As used herein, "neoangiogenic action (activity)" refers to the ability of a factor to form a new blood vessel in a target when acting on the target.

As used herein, "neurite outgrowth" refers to the outgrowth of any neurite, and is confirmed by observing if substantial outgrowth of a neurite is present using a microscope or the like.

As used herein, "synapse" refers to the conjugation between neurons or between a neuron and other cells (synaptic connection) or between conjugation sites. Synapse may be observed using an electron microscope. For example, the main features defining a synapse may be (1) the synaptic vesicle, (2) the synaptic cleft, (3) the thickening of presynaptic membrane and subsynaptic membrane (post-synaptic membrane) and the like. Therefore, as used herein, "synaptogenesis" refers to formation of a synapse in a position without synapse. The formation of a synapse can be confirmed by verifying the presence of synapse using the above method.

As used herein, "Hemagglutinating virus of Japan" and "HVJ" refer to virus belonging to *Paramyxoviridae* or Paramyxovirus having a cellular fusion action and are interchangeably used. M. Kuroya et al. (1953) reported the virus as Hemagglutinating virus of Japan. A genome therof is a minus-strand RNA consisting of approximately 15500 bases. Viral particle of Hemagglutinating virus of Japan has an envelope, and is polymorphic with a diameter of 150-300nm. Hemagglutinating virus of Japan has RNA polymerase. This virus is unstable under heat. It aggregates almost every type of erythrocytes and is hemolytic. It proliferates in an embryonated egg and/or cytoplasm of a cultured cell derived from a kidney of various animals. Hemagglutinating virus of Japan tends to cause persistent infection when it infects an established cell. Since it has the ability of fusing various cells, it is broadly used for cellular fusion such as for the formation of heterokaryon and the preparation of a hybrid cell.

As used herein, "(viral) envelope" refers to a membrane structure based on a lipid bilayer surrounding a nucleocapsid present in a specific virus such as Hemagglutinating virus of Japan. An envelope is typically found in viruses which mature by budding from a cell. An envelope generally consists of a small projection structure consisting of spike protein encoded by a viral gene and lipids derived from a host. Therefore, "(viral) envelope vector" is a term used in the case where an envelope is used as a vector (carrier), and it may be interchangeably used herein with (viral) envelope in some cases.

Transfer of a gene (that is, a cellular growth factor of the present invention in the form of nucleic acid) to the central nervous system (CNS) is achieved using various viral vectors including adeno-associated virus (AAV) (Fan D, et al., Neurosci Lett. 1998;248:61-4), retrovirus, (Franceschini IA, et al., J Neurosci Res. 2001;65:208-19), adenovirus (Miyaguchi K, Maeda Y, Colin C, Sihag RK., Brain Res Bull. 2000; 51:195-202) and herpes simplex virus (Johnson PA, Yoshida K, Gage FH, Friedmann T., Brain Res Mol Brain Res. 1992;12:95-102). These vectors have advantages and disadvantages with regard to human gene therapy. Although these methods are effective for gene transfer to the central nervous system in vivo, many problems such as safety and the amount transferred are found in gene therapy, particularly in gene therapy for humans. HVJ-E vector is effective in transfecting a gene to the central nervous system without any apparent toxicity. Hence, HVJ-E vector may be used in the present invention.

In order to solve these problems, the present inventors developed and used HJV (Hemagglutinating Virus of Japan)-envelope vector which is a nonviral vector, as described above. This vector system was developed based on the first-generation HVJ-based gene transfer using a viral envelope and liposome (HVJ liposome method, Yamada K, et al., Am J Physiol. 1996;271:R1212-20; Kaneda Y, et al., Exp Cell Res. 1987;173:56-69). The first-generation HVJ vector has great potential with regard to transfection to the central nervous system of rat and *Primate* (Yamada K. et al. supra; Hagihara Y, et al., Gene Ther. 2000;7:759-63) but has theoretical disadvantages such as complicated procedures and difficulty in preservation. HVJ-envelope (HVJ-E) vector, which is a novel nonviral vector system, uses only an envelope of HVJ to transfer exogenous gene.

As used herein, "inactivation" refers to inactivation of a genome, when it is used with regard to virus (for example, Hemagglutinating virus of Japan). Inactivated virus is replication-defective virus. Inactivation is achieved by a method described herein (for example, alkylation and the like). Examples of such a method for inactivation include, but not limited to, a method including the steps of: (a) inactivating virus (for example, HVJ) by treatment with alkylating agent; (b) obtaining concentrate of the virus or inactivated virus; and (c) purifying the virus or inactivated virus by column chromatography and subsequent ultrafiltration, and a method in which the order of these steps are changed.

As used herein, "alkylation" refers to substitution of hydrogen atom of an organic compound with an alkyl group. "Alkylating agent" refers to a compound which provides an alkyl group. Examples of alkylating agent include alkyl halide, dialkyl sulfate, alkyl sulfonate, dialkylzinc. Examples of preferred alkylating agent include, but are not limited to, β-propiolactone, butyrolactone, methyl iodide, ethyl iodide, propyl iodite, methyl bromide, ethyl bromide, propyl bromide, dimethyl sulfate, diethyl sulfate and the like.

According to one aspect, the present invention also relates to a method or therapy for the amelioration of cerebral functions or prevention of deterioration of cerebral functions.

According to another aspect, the present invention relates to a method or therapy for enhancing neurite outgrowth or synaptogenesis (a subject is particularly, but not limited to, those caused in a region of cerebral infarction or therearound).

Administration of a pharmaceutical composition and a medicament of the present invention is achieved orally or parenterally. Examples of parenteral delivery method includes local administration, intraarterial administration (for example, via carotid artery), intramuscular administration, subcutaneous administration, intramedullary administration, administration to a subarachnoid space, intraventricular administration, intravenous administration, intraperitoneal administration, intranasal administration and the like. In the present invention, any route may be used as long as the route delivers the composition to a section to be treated.

As used herein, "gene therapy" or "gene treatment" refers to a method for therapy of diseases by introduction of a nucleic acid (forexample, DNA) to a patient. While gene therapy includes a method using the step of injecting a naked nucleic acid, a vector can be used in many cases. In the present invention, a viral envelope is used as a vector. Gene therapy is performed by administration of an expressed or expressible nucleic acid to a subject. In such an embodiment of the present invention, a nucleic acid produces a protein encoded by the nucleic acid, and such a protein mediates therapeutic effects.

Any method for gene therapy used in the field of the art may be used in accordance with the present invention. Exemplary methods are described in common general information manuals such as: Goldspie L et al., Clinical Pharmacy 12:488-505 (1993); Wu and Wu, Biotherapy 3:87-95 (1991); Tolstoshev, Ann. Rev. Pharmacol. Toxicol. 32:573-596 (1993); Mulligan, Science260:926-932(1993); and Morgan and Anderson, Ann. Rev. Biochem. 62:191-217 (1993); May, TIBTECH 11(5):155-215 (1993). Generally known DNA recombination techniques are described in: Ausubel et al. (ed.), Current Protocols in Molecular Biology, John Wiley & Sons, NY (1993); and Kriegler, Gene Transfer and Expression, A Laboratory Manual, Stockton Press, NY (1990).

While a viral envelope may be used for the introduction in one embodiment of the present invention, a composition and a medicament of the present invention may contain, in addition to viral envelope, an appropriate, pharmaceutically acceptable carrier including an excipient or other compounds to enhance processing of the envelope, for preparing a pharmaceutically usable preparation.

As used herein, "pharmaceutically acceptable carrier" refers to a substance used in the production of a medicament or veterinary medicine and has no harmful influences on active ingredients. Examples of such a pharmaceutically acceptable carrier include, but not limited to, an emulsifier, a suspending agent, a solvent, an extender, a buffer, a delivery vehicle, a diluent, an excipient and/or a pharmaceutical adjuvant.

A pharmaceutical composition and a medicament of the present invention may be administered in any aseptic and biocompatible pharmaceutical carrier (including, but not limited to, saline, buffered saline solution and water) . Any of these molecules may be administered to a patient independently or in combination with other agents, in a pharmaceutical composition mixed with an adjuvant and/or pharmaceutically acceptable carrier. In one embodiment of the present invention, a pharmaceutically acceptable carrier is pharmaceutically inactive.

A pharmaceutical preparation for parenteral administration may be an aqueous solution of an active compound. For injection, a pharmaceutical composition of the present invention may be formulated in an aqueous solution, preferably a physiologically adaptable buffer solution such as Hanks' solution, Ringer's solution or buffered saline solution. Further, a suspended active compound may contain a liposome.

A pharmaceutical composition of the present invention encompasses a composition containing an effective amount of envelope of the present invention for achieving a desired purpose. "Therapeutically effective amount" or "pharmacologically effective amount" is a term sufficiently recognized by those skilled in the art, and refers to an amount of an agent effective for causing a desired pharmacological result. Therefore, a therapeutically effective is an amount sufficient for alleviation of symptoms of a disease to be treated. One useful assay for confirming the effective amount for a predetermined application (for example, therapeutically effective amount) is to measure the degree of recovery from a disease of interest. An actual dose depends on an individual to which a treatment is applied, and is preferably an amount optimized so that a desired effect may be achieved without causing any significant side effects. Determination of a therapeutically effective amount is sufficiently within the ability of those skilled in the art.

For any compound, a therapeutically effective amount may be initially estimated using a cell culture assay or any suitable animal model. Animal models may also be used for determining the desired range of concentration and administration route. Subsequently, a dose and route useful for administration to a human can be determined using such information.

A therapeutically effective amount refers to the amount of envelope to alleviate the degree or state of diseases. Therapeutic effects and toxicity of a compound may be determined based on cell culture or standard pharmaceutical procedures (for example, ED₅₀ (therapeutically effective dose for 50% of a group) and LD₅₀ (lethal dose for 50% of a group). The dose ratio between therapeutic effects and toxic effects is the therapeutic index, and is represented by a ratio of ED₅₀/LD₅₀. Pharmaceutical compositions with a great therapeutic index are preferred. Data obtained from cell culture assays and animal experiments are used for formulating a range of amount of composition for use in a human.
Such a dose of a compound is preferably within the range of circulating concentration containing ED₅₀ without little or no toxicity. Such a dose varies according to the administration form to be used, susceptibility of a patient and the administration route. For example, dose of an envelope is appropriately selected depending on conditions such as the age of the patient, the type of disease, type of envelope to be used, and the like.

In the case of administration of a cellular growth factor to a human using an envelope vector of the present invention, an envelope vector corresponding to 400-400,000HAU, preferably 1,200-120,000HAU, more preferably 4,000-40,000HAU, may be administered to one subject. An amount of exogenous gene contained in the envelope to be administered may be 2-2,000µg, preferably 6-600µg, more preferably 20-200µg, per subject.

As used herein, "HAU" refers to the activity of virus capable of aggregating 0.5% of chicken erythrocytes. 1HAU corresponds to almost 24,000,000 viral particles (Okada, Y. et al., Biken Journal 4, 209-213, 1961). The above-mentioned amount may be administered once or several times per day.

An exact amount is selected by individual clinician in view of the patient to be treated. Dose and administration is adjusted so as to provide sufficient level of active parts or desired effects. Another factor which may be considered is the severity of a state of disease (for example, size and position of a tumor; age, body weight, sex of a patient; dietary restriction, frequency, combination of drugs and susceptibility to reaction and resistance/response to therapy in administration). Depending on the half-life and clearance speed of a specific preparation, a pharmaceutical composition with prolonged action may be administered every three or four days, every week, once per two weeks or once per month. Guidance with regard to a specific dose and method of delivery is provided in known literatures in the field of the art.

An amount of a composition and medicament used in a method of the present invention can be readily determined by those skilled in the art by taking into consideration a purpose of use, subject patient (type, severity and the like), age, body weight, sex, case history of the subject, form or type of cytophysiologically active substance, form or type of a cell and the like.

Frequency of application of a method of the present invention to a subject (or a patient) can also be determined readily by those skilled in the art by taking into consideration a purpose of use, a disease to be treated (type, severity and the like), age, body weight, sex, case history and therapeutic progress of the patient. Frequency may be, for example, once per day to once per several months (for example, once per week to once per month). It is preferred to administer once per week to once per month while observing progress.

While a composition and medicament of the present invention may be used for application to a human, they may also be used for other hosts (for example, mammals and the like).

Molecular biologicalmethod,biochemicalmethod and microbiological method used herein are well known and commonly used in the field of the art, and are described in, for example, Ausubel F. A. et al. Ed. (1998), Current Protocols in Molecular Biology, Wiley, New York, NY; Sambrook et al. (1987), Molecular Cloning: A Laboratory Manual, 2nd Ed. , Cold Spring Harbor Laboratory Press, Cold Spring Harbor, NY; Experimental Medicine, Supplement, "Idenshidonyu & Hatsugen Kaiseki Jikkenho (Experimental Method for Analysis of Gene Introduction & Expression), YODOSHA CO., LTD. (1997) and the like.

According to the present invention, a method for delivering a cellular growth factor to the brain, includes the steps of: 1) temporarily occluding an artery of the head or cervix; and 2) introducing a cellular growth factor to the brain while the artery of the head or cervix is occluded. Here, by temporal occlusion of the artery, a cellular growth factor is effectively (for example, over twice) introduced to the brain. Examples of a method for temporal occlusion include balloon catheter, clipping and the like, and cerebral infarction as a physiological method. Balloon catheter and clipping is preferred. Temporal here means time period sufficient for the administration of a cellular growth factor (for example, at least one min., at least five min. or the like). A preferred time period may be 1-120 min.

The present invention relates to use in the production of a medicament for the treatment of disorder of cerebral functions. In such a use, any embodiments described herein may be used as a preferred embodiment of cellular growth factor.

Although content of DNA in a preparation can be appropriately adjusted depending on the disease to be treated, age and body weight of a patient and the like, it is typically 0.0001-100mg, preferably 0.001-10mg, for a DNA of the present invention. Such a preparation is preferably administered once per several days or several months.

### (Description of preferred embodiments)

Hereinafter, the description of preferred embodiments will be described. It should be understood that these embodiments are for illustration of the present invention, and the scope of the present invention is not limited to such preferred embodiments.

The present invention has revealed for the first time that cellular growth factors such as HGF, VEGF, FGF and the like ameliorates or prevents disorder of cerebral functions. Although results with regard to physiological phenomenon of brain at the level of blood vessel and cells have been conventionally shown, it is revealed for the first time in the present invention that cerebral function (particularly, memory functions and the like) is ameliorated as the entire system. Thus, the present invention has demonstrated amelioration or prevention at the level of function by the introduction of HGF or VEGF. Accordingly, HGF and VEGF are effectively used as an agent for therapy or prevention of various declines or disorders of cerebral functions, such as the decline or disorder of cerebral functions due to cerebral ischemia, reduction in cerebral blood flow and the like.

As a specific example, the present invention is effectively used as an agent for therapy or prevention of the decline or disorder of cerebral functions due to cerebrovascular occlusion, cerebral infarction, cerebral thrombosis, cerebral embolism, cerebral apoplexy (including subarachnoid hemorrhage, transient cerebral ischemia, cerebral arteriosclerosis and the like), cerebral hemorrhage, occlusive disease of the circle of Willis, injury in the head, cerebrovascular dementia, Alzheimer's dementia, sequela of cerebral hemorrhage or cerebral infarction, and the like.

Further, the present invention can also be used as an agent for therapy or prevention of the decline or disorder of cerebral functions due to neurodegenerative diseases such as Alzheimer's disease, senile dementia of Alzheimer type, amyotrophic lateral sclerosis, Parkinson's disease or the like.

According to the present invention, HGF, VEGF, FGF and the like can be respectively used independently or in combination. They can also be used with a gene of other vascular endothelial growth factors. Further, it is also possible to use HGF, VEGF, FGF and the like in the form of nucleic acid and proteins and the like in combination. Preferred combination is a combination of HGF in the form of nucleic acid and HGF protein or VEGF in the form of nucleic acid and VEGF protein. Combination of HGF in the form of nucleic acid and HGF protein is more preferable.

HGF protein used herein may be prepared by any method, as long as the protein has been purified so that it can be used as a medicament, and a commercially available product (for example, TOYOBO CO., LTD., Code No. HGF-101 and the like) may also be used. The cDNA of HGF obtained by the above cloning is inserted into an appropriate expression vector, and the expression vector is introduced into a host cell to obtain a transformant, thereby obtaining a recombinant HGF protein of interest from the culture supernatant of the transformant (see, for example, Nature, 342,440 (1989), Japanese Patent No. 2577091). VEGF protein can also be obtained in the same manner.

FGF may be those available from Kaken Pharmaceutical Co., Ltd., or may be prepared based on the sequences obtained from GenBank.

Next, specific examples of a method of gene transfer, form of transfer, dose to be transferred used in gene therapy of the present invention will be described.

When a composition containing a growth factor like HGF in the form of nucleic acid, such as an expression vector containing cDNA of HGF in an expressible form is administered to a patient, administration form of such a composition is generally divided into two groups: the case of using a nonviral vector; and the case of using a viral vector. A method for preparing and administering such an expression vector is explained in detail in manuals of experimentation and the like (Experimental Medicine, Supplement, Idenshichiryo no Kisogijutsu (Basic Techniques of Gene Therapy), YODOSHA CO., LTD. (1996), Experimental Medicine, Supplement, Idenshidonyu & Hatsugen Kaiseki Jikkenho (Experimental Method for Analysis of Gene Introduction & Expression), YODOSHA CO., LTD. (1997), Japan Society of Gene Therapy Ed., Idenshichiryo Kaihatsu Kenkyu Handobukku (Handbook for Developments and Researches of Gene Therapy), NTS (1999)). Hereinafter, the methods will be specifically explained.

A. In the case of using a nonviral vector Using a recombinant expression vector obtained by incorporating a gene of interest into a commonly used gene expression vector, the gene of interest can be introduced into a cell or tissue by the following method.

Examples of a method for gene introduction into a cell include lipofection, phosphoric acid-calcium coprecipitation, DEAE-dextran method, direct injection of DNA using a minute glass tube and the like. As a method for Examples of a method for gene introduction into a tissue include gene transfer using internal type liposome, gene introduction using electrostatic type liposome, HVJ-liposome method, improved HVJ-liposome method (HVJ-AVE liposome method), gene introduction mediated by a receptor, transfer of a DNA molecule with a carrier (metal particle) to a cell using a particle gun, direct introduction of naked-DNA, introduction using positively-charged polymer and the like. Applying any of these methods, a recombinant expression vector can be incorporated into a cell.

Among these methods, in the HVJ-liposome method, a DNA is encapsulated in a liposome formed by lipid bilayer, and then the liposome is fused with inactivated Hemagglutinating virus of Japan (HVJ). The HVJ-liposome method is characterized such that fusion activity with a cell membrane is higher than that in a conventional liposome method, and hence, is a preferred form of gene introduction. A method for preparing an HVJ-liposome is described in detail in literatures (Experimental Medicine, Supplement, Idenshichiryo no Kisogijutsu (Basic Techniques of Gene Therapy), YODOSHA CO., LTD. (1996), Experimental Medicine, Supplement, Idenshidonyu & Hatsugen Kaiseki Jikkenho (Experimental Method for Analysis of Gene Introduction & Expression), YODOSHA CO., LTD. (1997), J. Clin. Invest. 93, 1458-1464 (1994), Am. J. Physiol. 271, R1212-1220 (1996)), and is also described in detail in the examples below. Although Z strain (available from ATCC) is preferred as HVJ, basically, other HVJ strains (for example, ATCC VR-907, ATCC VR-105 and the like) can also be used.

Further, direct injection of naked-DNA is the simplest method of all the above methods, and in view of this, it is a preferred introduction method.

As an expression vector used here, any expression vector may be used, as long as it is capable of expressing a gene of interest in vivo. For example, pCAGGS (Gene 108, 193-200 (1991)), pBK-CMV, pcDNA3.1, pZeoSV (Invitrogen and Stratagene) may be used.

B. In the case of using a viral vector As a viral vector, viral vector such as recombinant adenovirus, retrovirus and the like is used in representative methods. More specifically, a gene of interest is introduced into, for example, a DNA virus or RNA virus such as detoxicated retrovirus, adenovirus, adeno-associated virus, herpesvirus, vaccinia virus, poxvirus, poliovirus, Sindbis virus, Hemagglutinating virus of Japan, SV40, immunodeficiency virus (HIV) and the like, and the cell is infected with the virus, thereby introducing the gene into the cell.

Examples of a method for introducing a gene therapy agent into a patient include an in vivo method in which a gene therapy agent is introduced directly into a living body, and an ex vivo method in which a certain type of cell is extracted from a human, and a gene therapy agent is introduced into the cell outside the body, followed by returning the cell into the body ( Nikkei Science (Japanese version of Scientific American), April, 1994, pp. 20-45, The Pharmaceuticals Monthly, 36(1), 23-48 (1994), Experimental Medicine, Extra, 12(15) (1994), Japan Society of Gene Therapy Ed. , Idenshichiryo Kaihatsu Kenkyu Handobukku (Handbook for Developments and Researches of Gene Therapy), NTS (1999)). In the present invention, the in vivo method is preferred.

As an administration site, an appropriate administration site is selected based on the disease to be treated, symptoms and the like. For example, in addition to the method in which the skull is directly perforated and a gene is introduced therein, administration to the lateral ventricle, administration to the subarachnoid space or intracisternal administration and the like are also included. Among them, administration to the subarachnoid space is an efficient administration method disclosed in the present invention. For the object of the present invention, that is, for the therapy of reducing cerebral blood flow by neoangiogenesis and/or by suppression of cerebral neurocyte death,administration to the subarachnoid space is preferred.

As a form of preparation, various forms of preparation respectively suitable for the above administration form (for example, liquid preparation or the like) may be taken. For example, in the case of an injection containing a gene as an active ingredient, such an injection can be prepared by an ordinary method. For example, it can be prepared by dissolving the active ingredient in an appropriate solvent (buffer solution such as PBS, saline, sterilized water and the like), filtration sterilization using a filter or the like, if necessary, and then filling the solution into an aseptic container. A commonly used carrier or the like may be added to such an injection, if necessary. Further, a liposome such as HVJ-liposome can be in the form of suspensions, frozen formulations, centrifugation-concentrated frozen formulations, and the like.

Further, for availability of a gene around a diseased site, it is also possible to prepare a sustained-release preparation (mini pellet formulations or the like) to be embedded close to the diseased part, or is continuously and gradually administered to the diseased site using an osmotic pump or the like.

Thus, according to one aspect, the present invention provides a composition for the prevention of deterioration of cerebral functions or amelioration of cerebral functions, which contains a cellular growth factor. Although it has been shown that a cellular growth factor supports physical or external amelioration of cerebral vasculature and the like, actual amelioration of functions has not been reported. Further, it is not recognized that functions of the nervous network can be ameliorated by mere external amelioration (for example, neoangiogenesis), and the relationship between the ameliorations is also unclear. Therefore, even if neoangiogenesis caused by a factor is observed, it is not expected that the neoangiogenesis is effective for amelioration of the functions or prevention of deterioration of the functions until amelioration of the functions is actually demonstrated. It is thus recognized that the present invention attains significant effects unexpected by those skilled in the art. The present invention achieves effects of amelioration of cerebral functions or prevention of deterioration of cerebral functions, which could not be achieved conventionally. In the present invention, administration of a cellular growth factor such as HGF to the brain attained amelioration of functions in the Morris water maze test.

According to another aspect, the present invention provides a composition for enhancing neurite outgrowth or synaptogenesis, which contains a cellular growth factor as an active ingredient. Although it has been shown that a cellular growth factor supports physical or external amelioration of the cerebral vasculature and the like, enhancement of neurite outgrowth or synaptogenesis has not been reported. Further, it is not recognized that functions of the nervous network can be ameliorated by mere external amelioration (for example, neoangiogenesis), and the relationship between the ameliorations is also unclear. Therefore, even if neoangiogenesis by a factor is observed, it is not expected for neoangiogenesis to be effective for the amelioration of the functions or prevention of deterioration of the functions until enhancement of neurite outgrowth or synaptogenesis is actually demonstrated. It is thus recognized that the present invention attains significant effects unexpected by those skilled in the art. The present invention achieves effects of enhancement of neurite outgrowth or synaptogenesis, which could not be achieved conventionally. In the present invention, it was found that administration of a cellular growth factor such as HGF to the brain enhanced neurite outgrowth or synaptogenesis.

In one embodiment, a cellular growth factor used in the present invention preferably has action of inducing vascular growth. Examples of such a cellular growth factor representatively include VEGF, FGF, HGF and the like. More preferably, HGF is used.

Such a cellular growth factor may be administered with a viral envelope. Here, while any viral envelope can be used, an inactivated viral envelope is preferably used. While any type of viral envelope may be possible, an envelope of RNA virus may be preferably used. Here, such an viral envelope is preferably an envelope of *Paramyxoviridae* virus, and more preferably, an envelope of HVJ (hemagglutinating virus of Japan). This is because such an envelope achieves efficient gene introduction and thus is more suitable for gene therapy, although restriction to the theory is not intended. However, it is understood that the present invention is not limited to these envelopes.

In one embodiment, a cellular growth factor used in the present invention is provided in the form contained in a viral envelope.

Cerebral functions of interest in relation to a composition or method of the present invention for which amelioration or prevention of deterioration is intended include cognitive function. Among cerebral functions, representative examples of such a cerebral function include a cerebral function affected by disorder of cognitive function or motor function. Examples of such a disorder include cerebral infarction, disorders caused by cerebral blood flow disorder, cerebral hemorrhage or cerebrovascular disorder which results in cognitive dysfunction or motor dysfunction.

In another embodiment, cerebral functions of interest of the present invention include memory function and spatial learning function. In particular, almost no drug characterized by amelioration of a function selected from the group consisting of memory function and spatial learning function has been known so far, and thus the superiority the present invention should be noted. Although restriction to the theory is not intended, amelioration of such a cerebral function may be achieved by the activation of astrocytes, but not limited thereto.

While a cellular growth factor used in the present invention may be administered in any form, as long as the factor eventually attains the function in the brain, it may be representatively provided in the form of a protein or nucleic acid. When a cellular growth factor is administered in the form of nucleic acid, such a form is referred to as a form of gene therapy.

While a composition of the present invention may be administered through any route as long as the composition is delivered to the brain and achieves amelioration of functions or prevention of deterioration of functions, for example, administration to the subarachnoid space or intracisternal administration is preferred.

One of the features of the present invention is that effects of ameliorating functions were observed even after a long period (for example, over one day, two days, three days, four days, five days, six days, seven days and the like) from the occurrence of disorder of cerebral functions. Thus, the effects of the present invention should be appreciated in that no urgency such as within several hours from the occurrence of disorder is required. In one preferred embodiment, for example, a composition is administered after six days from the development of disorder of cerebral functions (in case where the day of development is counted as day 1, after day 7).

In particular, in one embodiment, the present invention attains effects as a form of gene therapy, even when a cellular growth factor used is in the form of nucleic acid and is administered after six days from the development of disorder of cerebral functions.

According to another aspect, the present invention provides a method for the prevention of deterioration of a cerebral function or amelioration of a cerebral function, which includes the step of: (A) administering a cellular growth factor to a patient. It will be understood that a patient as a subject of the present invention may be any organism as long as the organism (for example, mammals such as Rodentia, Primates and the like) has cerebral functions (higher functions such as memory function or learning function, for example). A subject is preferably primates including human, and is more preferably a human. The present invention has great significance as it proved for the first time that a cellular growth factor is effective for an application for amelioration of cerebral functions or prevention of deterioration of cerebral functions.

Alternatively, according to another aspect, the present invention provides a method for enhancing neurite outgrowth or synaptogenesis, which includes the step of: (A) administering a cellular growth factor to an individual for which enhancement of the neurite outgrowth or synaptogenesis is necessary. It will be understood that a patient as a subject of the present invention may be any organism as long as the organism (for example, mammals such as Rodentia, Primates and the like) has a nervous system (system in which neurite outgrowth or synaptogenesis can be observed). A subject is preferably primates including human, and is more preferably a human. The present invention has great significance as it proved for the first time that a cellular growth factor is effective for an application as an enhancement for neurite outgrowth or synaptogenesis in a cerebral infarction region or therearound.

It will be understood that a cellular growth factor used in this method of the present invention may take a form of any composition as explained herein.

According to another aspect, the present invention provides a use of a cellular growth factor in the production of a medicament for the prevention of deterioration of a cerebral function or amelioration of a cerebral function. It will be understood that a cellular growth factor used in this use of the present invention may take a form of any composition explained herein. As such, a cellular growth factor, a vascular endothelial growth factor (VEGF), a fibroblast growth factor (FGF), a hepatocyte growth factor (HGF) and the like can be preferably used. A hepatocyte growth factor (HGF) can be used most preferably.

Alternatively, according to another aspect, the present invention provides a use of a cellular growth factor in the production of a medicament for the enhancement of neurite outgrowth or synaptogenesis. It will be understood that a cellular growth factor used in this use of the present invention may take a form of any composition explained herein. As such, a cellular growth factor, a vascular endothelial growth factor (VEGF), a fibroblast growth factor (FGF), a hepatocyte growth factor (HGF) and the like can be preferably used. A hepatocyte growth factor (HGF) can be used most preferably.

### (Examples of administration method)

Next, examples of a method for the actual administration of a gene will be explained. It will be understood that, while the present invention may be implemented as follows, experimentation or administration may be performed in accordance with other embodiments.

### Experiment I: An exemplary experimental protocol

### Materials and experimental methods

### 1) Ligation of both sides of the carotid artery

For example, male Sprague Dawley rats (350-400g; Charles River Japan, Atsugi, Japan) were anesthetized with pentobarbital sodium (50mg/kg, interperitoneally), thereby allowing natural respiration by the rats. Both sides of the carotid artery were exposed by midline incision in the cervix, and could be firmly ligated with a 2-0 silk.

### 2) Preparation of HVJ-liposome complex

As a method used for the preparation of HVJ-liposome complex, a technique described in literatures (J. Clin. Invest. 93, 1458-1464 (1994), Am. J. Physiol. 271, R1212-1220 (1996)) can be used. Briefly, phosphatidylserine, phosphatidylcholine and cholesterol can be mixed in a weight ratio of 1:4.8:2. This lipid mixture (10mg) can be precipitated on a side surface of a flask by removing tetrahydrofuran in a rotary evaporator. Dried lipids can be hydrated in 200µl of balanced salt solution (BSS; 137µM NaCl, 5.4µM KCl and 10µM Tris-HCl (pH 7.6)) containing an expression vector in which a gene of interest has been inserted. Liposomes for the control group contains an expression vector in which a gene of interest has not been inserted (BSS, 200µl). Liposomes can be prepared by shaking and ultrasonic treatment of the mixture.

Purified HVJ (Z strain) was inactivated immediately before use thereof by UV irradiation for three minutes (110 erg/mm²/sec.). The liposome suspension (0.5ml, containing 10mg of lipids) can be mixed with HVJ (10,000 HAU in BSS of a total amount of 4ml). This mixture was incubated at 4°C for five min., and was then incubated at 37°C for 30min. while gently shaken. Free HVJ was removed from the HVJ-liposome by sucrose density-gradient centrifugation. Top layer of the sucrose gradient can be recovered and used. Final concentration of plasmid DNA was equal to 20µg/ml as evaluated in accordance with a previous report (J. Clin. Invest. 93, 1458-1464 (1994), Am. J. Physiol. 271, R1212-1220 (1996)). This preparation method is optimized so as to achieve the maximum transfection efficiency.

3) Gene introduction in vivo In order to establish an efficient gene introduction method, we can test three different methods for delivering plasmid which forms a complex with the HVJ-liposome, that is: 1) direct injection into the internal carotid artery; 2) injection into the lateral ventricle; and 3) intracisternally injection (to the subarachnoid space).

In the injection into the internal carotid artery, male Sprague Dawley rats (350-400g) were anesthetized with pentobarbital sodium (50mg/kg, intraabdominally), and the left common carotid artery were then incised. Thus, a polyethylene catheter (PE-50, ClayAdams, Parsippany, New Jersey) can be introduced (Rakugi and the like). Distal part of the external carotid artery was separated with a provisional ligature for a short time. The HVJ-liposome complex (1ml) was then injected into the area of the external carotid artery. After injection, the injecting cannula was removed, and then the ligature was loosened, thereby recovering blood flow to the common carotid artery.

In the injection into the lateral ventricle, anesthetized rats can be laid on a stereotaxic frame (Narishige Scientific Instrument Laboratory, Tokyo, Japan), thereby exposing the skull. Specifically designed stainless steel cannula (30 gauge; Becton Dickinson, Franklin Lakes, New Jersey) having a Teflon (registered trademark) connector (FEP tube, Bioanalytical Systems, West Lafayette, Indiana) can be introduced into the left lateral ventricle in a manner described in Am. J. Physiol. 271, R1212-1220 (1996). Stereotaxic coordinate can be determined as follows: 1.3mm behind bregma, 2.1mm lateral to midline and 3.6mm under skull surface. The HVJ-liposome complex was injected into the lateral ventricle (20µl). After injection, the injecting cannula can be removed. It was observed that behavioral change such as convulsion of the extremities or abnormal behavior was not observed in any of the animals subjected to injection.

In injection into a subarachnoid space, each animal was fixed by the head in the decubitus, and the atlantooccipital membranes were exposed by midline incision of the occipital bone. A stainless steel cannula (27 gauge; Becton Dickinson, Franklin Lakes, New Jersey) was introduced to the subarachnoid space. The position of the cannula was confirmed. After removing 100µl of cerebral spinal fluid in order to avoid an increase in the intracerebral pressure, the HVJ-liposome solution (100µl:100µg/ml) was carefully injected into the cisterna magna (subarachnoid space) over one minute. Thereafter, the animals were laid with their head down for 30 min. The aseptic procedure can then be completed after administration of antibiotic in a preventive dose (30,000U of penicillin G).

### 4) Record

For example, using a laser Doppler imager (LDI) for laser Doppler imaging, blood flow can be continuously measured and recorded for two postoperative weeks. In an LDI system (Moore Instruments Ltd., Devon, GB), 2mW helium-neon laser is incorporated in order to cause a ray to continuously scan the surface of a tissue in a size of 12 × 12cm to a depth of 600µm. During scanning, hemocytes moving in a vasculature change the vibration frequency of incident light in accordance with the Doppler theory. Since a photodiode concentrates diffuse light in a reverse direction, original variation in light intensity is converted into voltage variation in a range of 0-10V. Output perfusion value of 0V can be graduated as 0% perfusion, and 10V can be graduated as 100% perfusion. When scanning is completed and the diffuse light in a reverse direction is concentrated from all of the sites to be measured, an image showing the distribution of blood flow distinguished by different colors is displayed on a television monitor. The perfusion signal is divided into six different sections which are respectively displayed as different colors. A portion with reduction in blood flow or a portion with no perfusion is represented by dark blue, while a portion with the maximum perfusion is represented by red.

Using LDI, perfusion in the brain surface before occlusion, immediately after occlusion, on day 7 and day 14 after occlusion can be recorded. Through a midline incision portion on the scalp, bone window in a size of 12 × 12 can be formed with an electric drill. Continuous measurement value can be obtained using this bone window. An image distinguished using different colors is recorded, and then analysis is performed by calculating average perfusion value for each rat. In order to take parameters including ambient light and temperature into consideration, the calculated perfusion value can be represented as a ratio of post-treatment (ischemic) brain to pre-treatment (untreated) brain.

### 5) Histopathological test

After fixation in 3% paraformaldehyde/20% sucrose solution for one day, 25µm frozen section of the coronal plane can be produced from each 100µm section for X-gal staining. Staining with X-gal allows the identification of a stained neuron expressing β-galactosidase. For alkaline phosphatase staining (ALP), 25µm frozen section of coronal plane can be produced from each 100µm section. These sections are incubated with PBS containing 0.3% hydrogen peroxide to decrease intrinsic peroxidase activity, and then incubated with the first antibody or lectin diluted in PBS containing 10% equine serum at a room temperature for 60 min. After washing in Tris buffered saline solution containing 2% equine serum for three times, biotin-added second antibody suitable for the species and subsequently avidin-biotin peroxidase complex (Vectastain ABC kit, PK6100, Vector Laboratories, Burlingame, California) can be added and the solution can be incubated. Binding of the antibodies can be visualized using diaminobenxidine. It is possible to omit the first antibody and perform staining with an unrelated immunoglobulin which is suitable for the type and class of the antibody for use as a negative control for each antibody.

### 6) ELISA method for HGF and VEGF in cerebrospinal fluid (CSF)

CSF (100µl) obtained from rats before or 7 or 14 days after occlusion in both sides of the carotid artery were used in these experiments. Rat and human HGF was measured by ELISA kit (Institute of Immunology, Tokyo), and human VEGF can also be measured using ELISA kit (R & D systems, Minneapolis, Minnesota).

### 7) Experimental materials

Human HGF can be obtained for use by cloning human HGF cDNA (Japanese Patent No. 2577091) using an ordinary method and by inserting the clone into an expression vector, pcDNA (available from Invitrogen).

Human VEGF can be obtained for use by cloning human VEGF165 cDNA (Science 246, 1306 (1989)) using an ordinary method and by inserting the clone into an expression vector, pUC-CAGGS.

Human recombinant HGF can be prepared for use by transfecting ovarian cell of a Chinese hamster (ATCC) or C-127 cell (ATCC) with a recombinant expression vector obtained by inserting a human HGF cDNA (Japanese Patent No. 2577091) into an expression vector pcDNA (Invitrogen) and then purifying from the culture medium using an ordinary method.

Administration of the present invention can be performed based on, but not limited to, the above materials and experimental methods.

A patent, patent application, and reference cited herein is incorporated herein as reference of the present specification in its entirety as if the entirety of each literature were specifically described herein.

Although the present invention has been illustrated hereinabove by way of preferred embodiments of the invention, the invention should not be understood as limited to the embodiments. It will be understood that the scope of the present invention is interpreted as only depending on the claims. From the specific description of the preferred embodiments of the present invention, it will be understood that those skilled in the art can carry out the invention within an equivalent scope based on the description and common general knowledge. It will also be understood that a patent, patent application, and reference cited herein should be incorporated herein by reference of the present specification in its entirety as if the entirety per se were specifically described herein.

### EXAMPLES

Hereinafter, the constitution of the present invention will be explained in more detail. The present invention is not limited thereto. In the following examples, commercially available reagents are from TOYOBO CO., LTD. (Osaka, Japan) and the like, unless particularly mentioned otherwise. Mice and the like were obtained from CLEA Japan, Inc. (Kanagawa, Japan) and the like. The animals were treated in accordance with ethical regulations of Osaka University or ethical regulations recommended by the Japanese government.

### (Example 1: Amelioration of cerebral functions) (Method)

### (Preparation of the HVJ-envelope vector)

HVJ-envelope vector was prepared in accordance with a known method (Kaneda, Y. et al., Mol Ther 6, 219-26 (2002) and Shimamura, M. et al., Biochem Biophys Res Commun 300, 461-71 (2003)). Briefly, suspended virus (15000 hemag-glutination units) was inactivated by UV irradiation (99mj/cm²), and was mixed with plasmid DNA (400µg) and 0.3% Triton-X. After centrifugal separation, the resultant was washed in 1ml of balanced salt solution (BSS; 10mM Tris-Cl (pH7.5), 137mM NaCl and 5.4mM KC1), and thus the surfactant and DNA which were not incorporated were removed. After centrifugal separation, this envelope vector was suspended in 100µl of phosphate buffered saline (PBS). This vector was kept at 4°C until use thereof.

### (Construction of plasmid)

In order to prepare HGF expression vector, human HGF cDNA (2.2kb) was inserted into an eukaryotic expression plasmid using a cytomegalovirus (CMV) promoter/enhancer (Koike, H. et al., Faseb J5, 5 (2003)). Using this promoter/enhancer, reporter genes were expressed in various cell types. The expression can be regarded as constitutive expression. These control vectors were expression vector plasmids with the same structure which contained a promoter but did not contain HGF cDNA. The plasmids were purified using QIAGEN plasmid isolating kit (Qiagen, Hilden, Germany). The prepared sequences is set forth in SEQ ID NO:7 (pcDNA3.1(-)HGF) and SEQ ID NO:8 (pVAX1HGF/MGB1).

### (Surgical procedure)

Wistermale rats (270-300g; Charles River Japan, Atsugi, Japan) were used for this study. In order to produce permanent MCAo models, as described above, poly-L-lysine-coated 4-0 nylon was arranged around the MCA source, thereby occluding the right middle cerebral artery (Nelayev, L. et al., Stroke 27, 1616-22 (1996)). Briefly, the animals were anesthetized with halothane (1-3.5% in a mixture of 70% N₂O and 30% O₂) using a facial mask. Using a feedback-controlled heating pad, the temperature of the rectum and skull was maintained at 37±0.5°C throughout the surgical procedure (Unique Medical, Tokyo, Japan). Using an operating microscope, the right common carotid artery, right external carotid artery and right internal carotid artery were separated through median incision. 4-0 nylon was inserted from the right external carotid artery, and was inserted therein by 20mm. The right external carotid artery was then ligated using 6-0 nylon.

Based on a known method, in vivo gene transfer was performed by intercisternal injection (Shimamura, M. et al., 2003, supra). Briefly, the rats were anesthetized with ketamine (Sankyo, Japan) and xylazine (Bayer Ltd., Japan). Each of the animals was fixed by the head with their face down, and the atlantooccipital membrane thereof was exposed through the posterior median incision. A stainless cannula (27 gauge; Becton Dickinson) was then introduced into the cisterna magna (subarchnoid space). After removal of 100µl of cerebrospinal fluid (CSF), HJV-envelope vector (100µl) containing human HGF was injected at a speed of 50µl/min. The animals were then left with their heads down for 30 min.

### (Protocol of treatment and behavioral test)

Figure 1 shows the summary of the protocol of the behavioral test. 10 rats were merely anesthetized (sham operation) and 60 rats were exposed to MCAo (day 1). Based on the neuromuscular functions and body weights evaluated on day 7, these rats were equally divided into vehicle-treated rats (n=23) and HGF-treated rat (n=23). Rats which did not show paralytic symptom on day 7 or those that died by day 7 were excluded from the experiment (n=14). On day 55, alive rats (n=20 for vehicle-treated rats, and n=22 for HGF-treated rats) were evaluated for neuromuscular functions and spontaneous activity. Subsequently, they were tested for cognitive function by the MWM test and passive avoidance learning. On day 96, MRI was performed to evaluate the degree of infarction.

For histopathological analysis on day 14 and day 56, other rats (in the respective experimentations, vehicle-treated rats (n=4) or HGF-treated rats (n=4)) were treated in the same manner as described above, and were killed on day 14 and day 56.

### (Sensorimotor dysfunction)

Among various batteries for sensorimotor dysfunction, the present inventors used a simple protocol for evaluating sensorimotor dysfunction using the following categories (maximum score: 4) (Petullo, D. et al., Life Sci 64, 1099-108 (1999)). Bending of the forelimb: the rats were fixed by the tail on a flat surface. Paralysis of the forelimb was evaluated based on the degree of bending of the left forelimb. Twist of the body: the rats were fixed by the tail on a flat surface. The degree of torsion of the body was studied. Push to a side: the rats were pushed to the left or right side. Rats with right MCA occlusion showed either weakness with respect to the push to the left side or did not show any resistance. Rearrangement of the posterior limb: one of the posterior limbs was removed from the surface. When the limb was removed, rats with right MCA occlusion slowly rearranged or simply did not rearrange the posterior limb at all.

### (Spontaneous activity)

Spontaneous activity was measured through open field test for 30 min. using an automated activity box (Muromachi Kikai, Tokyo, Japan).

### (Morris water maze learning)

Water (25°C) was filled in a cylindrical tank with a diameter of 1. 5m, and a transparent platform with a diameter of 15cm was laid on a fixed position at the center of one of the four quadrants (O'HARA & CO., LTD., Tokyo, Japan). For adaptation before the test, the rats were made to swim freely for one min. In the invisible platform test, the platform was set under the surface of the water to prevent the rats from seeing the platform. The platform was fixed to one of the quadrants and the starting point was changed for each test. Previous studies show that, when a test is performed twice per day, no difference in the latent period for reaching the platform can be observed between the rats exposed to MCAo 12-14 weeks before and the control rats, until day 6 of the session (Modo, Met al., J Neurosci Methods 104, 99-109 (2000)). Based on these results, the present inventors performed the test twice per day for six days. When a rat could not reach the platform, the latent period was evaluated as 60 sec. In the visible platform test, a flag was put on the platform so as to be seen by the rats. This test was performed twice per day for six days. In this test, the platform and starting point were changed each time. Through these tests, the swimming routes were captured with CCD video camera, and were analyzed by NIH images.

### (Passive avoidance learning)

In this experimentation, step-through passive avoidance learning was used. An apparatus having a light chamber and a dark chamber (MEDICAL AGENT, Kyoto, Japan) was used. For adaptation, the rats were put into the light chamber, and the door was left open so that the rats could enter the dark chamber. Rats like dark places, and thus have a habit of going toward the dark chamber. In the acquisition trial, the rats were put into the light chamber, and when the rats entered the dark chamber, they were given a shock at 6.0mA. The respective tests were continued until the rats learn to keep out of the dark chamber for 300 sec. In the memory trial, the rats were placed in the light chamber one, three, five and seven days after the acquisition trial. The present inventors then evaluated the latent period (300 sec. to the maximum) during which the rats stayed in the light chamber.

### (Immunohistochemistry)

For immunohistochemistry, the rats were killed and transcardial perfusion fixation was performed in ordinary saline and then in 4% formaldehyde. The brain was extracted, fixed, subjected to cryoprotection treatment, and then sliced into 12µm or 30µm sections in a cryostat. After blocking, the section was incubated in 3% goat serum and anti-MAP2 antibody (1:1000, Sigma-Aldrich, Saint Louis, MO, USA), anti-GFAP antibody (1:1000, Sigma-Aldrich) and anti-Cdc4 antibody 2 (1:500, Santa Cruz, CA, USA). Thereafter, the section was incubated in an anti-murine fluorescent antibody (1:1000 for NAP2 and GFAP and 1:500 for Cdc42, Alexa Flour 488, Molecular Probes, USA).

For the immunohistochemistry for Cdc42 and synaptophysin in the infarction region and contralateral region, the following antibodies were used: anti-Cdc42 antibody (murine monoclonal antibody; Santa Cruz, CA, USA); and anti-synaptophysin antibody (murine monoclonal antibody; Chemicon, Temecula, CA, USA).

### (Quantitative analysis of immunohistochemistry)

In order to quantify immunoreactivity to GFAP, the obtained image was imported in Adobe Photoshop (version 7.0, Adobe Systems, San Jose, CA, USA). The color image was converted into a gray scale image. This gray scale image was imported in Mac SCOPE (version 2.5, MITANI CORPORATION, Fukui, Japan). ROI was determined as a region of cerebral cortex adjacent to an infarction region. The number of pixels with a signal equal to or higher than 25 was counted. The immunoreactivity was calculated using the following formula: % region= [number of pixels with a higher signal]/[total number of pixels].

### (ALP staining)

For ALP staining, the section was washed in Tris-HCl and then incubated in a substrate solution (mixture of AS-BI phosphate (Sigma-Aldrich) and fast red violet LB salt (Sigma-Aldrich)) for 30min.

Five adjacent sections of each rat were observed, and the obtained images were imported in Adobe Photoshop. The color images were converted into gray scale images. Subsequently, ROI was determined as a peri-infarcted region. The area and length of the blood vessel were analyzed using Angiogenesis Image Analyzer (version 1.0, KURABO, Tokyo, Japan).

### (Magnetic resonance image)

For morphological evaluation of a volume of ischemia, high resolution T2-weightedmagnetic resonance image (MRI) (2DFSE, TR=5,000msec., TE=102msec.) was obtained using a 3T MRI scanner (Sigma LX VAH/I, GE, Milwaukee, USA). The image was produced with a matrix of 256 × 256 × 21 and with a pixel size of 0.39 × 0.39 × 1.5mm.

### (Results)

In order to investigate the severity of cerebral infarction, all rats were observed using T2-weighted MRI on day 96 (Figure **1**). Although there was no difference in the total volume of infarction calculated from the T2-weighted image between the groups (Figure **2a**), patters of cerebral infarction were divided into three groups: group in which a region with high intensity is found in the cortex and basal ganglia (type A); group in which a region with high intensity is found in a part of the cortex and the basal ganglia (type B); and a group in which a region with high intensity is found in a smaller region (Figure **2b** and Figure **2c**). In order to eliminate the possibility of diversity of cognitive dysfunction based on the diversity of MCAo models, the present inventors paid attention to the rats of group A in the present study. The present inventors reevaluated the volume of infarction for the rats in group A (Figure 2d, n=15 (vehicle-treated rats), n=17 (HGF-treated rats)), but there was no significant difference between these groups.

Since sensorimotor dysfunction and spontaneous behavior influences the result of the test for cognitive function to some degree, the present inventors peroformed some tests for them (DeVries, A.C. et al., Neurosci Biobehav. Res. 25, 325-42 (2001)) . By day 55, sensorimotor dysfunction was recovered to some degree in both groups, and no difference was observed between the groups (Figure **3a)**. In the spontaneous activity test (DeVries, A.C. et al., Supra) for measuring spontaneous activity of the rats, as previously described (Robinson, R.G. et al. , Science 205, 707-10 (1979), the rats subjected to MCAo showed increased activity in comparison with the sham-treated rats, but there was no difference between vehicle-treated rats and HGF-treated rats (Figure **3b).**

Subsequently, the present inventors performed Morris water maze (MWM) test, thereby evaluating spatial learning and memory (DeVries, A. C. et al., Supra). Although all of the rats subjected to MCAo showed an increase in the latent period before reaching the goal in the MWM test, the latent period was gradually reduced for HGF-treated rats in the invisible platform test (Figure **4a**). In order to eliminate the possibility of influence of loss of vision, sensorimotor dysfunction that will have on the results (DeVries, A.C. et al., Supra), the present inventors also performed a visible platform test. For all of the rats subjected to MCAo, increased latent period was observed, but there was no significant differences between the vehicle-treated rats and HGF-treated rats (Figure **4b).** Throughout the test, no difference in swimming velocity was observed between the vehicle-treated rats and the HGF-treated rats. Accordingly, spatial learning and memory of the HGF-treated rats was partly, but significantly recovered.

In the passive avoidance learning used for measuring related learning (DeVries, A.C. et al., Supra), the rats subjected to MCAo required more stimulation for acquisition of this learning, and there was no difference between the vehicle-treated rats and HGF-treated rats (Figure **5a**). However, the latent period in the memory trial was significantly longer for the HGF-treated rats, around three days after the acquisition trial. This indicates that the HGF-treated rats may maintain memory longer than the vehicle-treated rats. Thus, it is revealed that the treatment method of the present invention achieves amelioration of cerebral functions.

Subsequently, the present inventors attempted to investigate the mechanism of recovery of functions by HGF therapy. For both groups, immunoreactivity to GFAP, a marker of astrocyte, increased in the peri-infarcted region on day 14 and day 56. The immunoreactivity of the HGF-treated rats were higher than the vehicle-treated rats on day 14 and lower on day 56 (Figure **6** and Figure **7a).** Although cells that are immunoreactive to MAP2, as a marker of matured neuron, decreased in the peri-infarcted region for both groups, there was no significant difference between the groups (Figure **7b**). As previously described, for all of the rats, imunoreactivity to Cdc42 (one of GTPase of Rho family with positive effects on neurite outgrowth) was observed in the hippocampus. Although the peri-infarcted region in the cerebral cortex showed immunoreactivity to Cdc42 for both groups (Figure **8a**), the number of cells immunoreactive to Cdc42 was significantly greater for the HGF-treated rats (Figure **8b).**

Arteries in the peri-infarcted region and contralateral region on day 14 and day 56 were investigated using ALP staining. In the perieinfarcted region, the number of arteries of the HGF-treated rats on day 56 was significantly increased (Figure **9).** Further, quantitative analysis showed that the arterial region increased and the arteries were elongated on day 56 (Figure **10).** Regarding the contralateral regions, there was no difference between the groups on day 14 and day 56 (Figure **10).**

Subsequently, on day 7 of cerebral infarction, a control gene and HGF gene was administered. On day 14 of cerebral infarction, the rats were killed, and immunostaining for Cdc42, which is a smallG protein expressed in neurite outgrowth, was performed. On day 56, immunostaining for synaptophysin expressing in a synapse was performed. In both cases, stainability of the HGF-administered group increased in the peri-infarcted regions (Figure **11).**

### (Discussion)

The present invention demonstrated that the results from MWM and passive avoidance learning were significantly preferable for the HGF-treated rat on day 56 and that the size and patterns of infarction did not change. Similar to the previous report (Dijkhuzen, R.M. et al., Supra, Zhang, L. et al., J Neurol. Sci. 174, 141-6 (2000)), sensorimotor dysfunction was spontaneously recovered for the rats subjected to MCAo, and there was no significant difference between the groups. Although some previous studies have showed that neurotrophic factor have useful effects on the recovery from sensorimotor dysfunction within one month after ischemic disorder (Kawamata, T et al., Proc. Natl. Acad. Sci. USA 94, 8179-84 (1997)), the disorder on day 56 in the present studies was not so severe as to show detectable difference. Further, there was no significant differences in the spontaneous activity test and the visible platform test in MWM. These results suggest that the results from MWM and the passive avoidance learning do not reflect the difference in sensorimotor dysfunction or spontaneous activity but difference in learning and memory (DeVries, A.C. et al., Supra). The present inventors consider that the obtained difference depends on functions of the peri-infarcted region in the cerebral cortex. This is because the fact that retention of memory was not observed in spatial learning and passive avoidance in MWM is related to damage to the cerebral cortex (Yonemori, F. et al., J. Cereb Blood Flow Metab. 19, 483-94 (1999), Hirakawa, M. et al., Stroke, 25, 2471-5 (1994)), and there was some histological difference in the peri-infarcted region of the cerebral cortex. Recent studies have shown that the recovery of function is principally related to protection and recovery of hemisphere infarction activity in the cortex (Dikhuiezen, R.M. et al., Supra), and the results from this study are also supported by the recent studies.

Recent reports have revealed that HGF and c-Met are upregulated in astrocytes mainly in the peri-infarcted region after 4-28 days of MCAo, and are related to repair of cerebral tissue after ischemia (Nagayama, T. et al.). From these viewpoints, the present inventors paid attention to the histological alteration in the peri-infarcted region. In the present invention, astrocyte was activated on day 14, but was inactivated on day 56. This is similar to a previous study which has shown effects of BDNF on ischemic disorders (Schabitz, W.R. et al., Stroke 35, 992-7 (2004)). In other words, the astrocyte was temporarily inactivated during the acute stage of the disorder. Initially, activation of astrocytes was believed to be a part of the formation of glial scar which inhibits growth of axon (Eng, L.F. et al., Prog Brain Res 71, 439-55 (1987), Silver, J. et al., Nat Rev. Neurosci. 5, 146-56 (2004)). However, another report has shown that a temporarily activated astrocyte may enhance neuronal survival and growth of axon (Albrecht, P. J. et al., Expo Neurol 173, 46-62 (2002)). The present inventors consider that exogenous HGF may temporarily activate an astrocyte, enhance the growth of the axon, and/or directly enhance the growth of axon, but such HGF may not cause proliferation of astrocytes to form a scar of astrocyte (Sun, W. et al., Supra). This discussion is supported by increased immunoreactivity to Cdc42, which has positive effects on neurite growth in neuronal cell (O'Kane, E.M. et al., Supra) in the peri-infarcted region. Regrettably, there has not been any report showing in vivo induction of Cdc42 in a neuronal cell by exogenous HGF, but it has been reported that HGF stimulation of endothelial cells leads to activation of Cdc42 with the formation of filopodia and lamellipodia (Royal, I. et al., Mol Biol Cell 11, 1709-25 (2000)). Although further studies are required to clarify the function of HGF in the interaction between astrocytes and axon growth, one possible function is that the reconstruction of neuronal network in the peri-infarcted region may lead to better effects for HGF-treated rats.

Another possible mechanism may be the influence of neoangiogenesis in the peri-infarcted region. Although it is still unknown whether or not the degree of neoangiogenesis is related to functional recovery, a recent report has demonstrated that collateral growth and new capillaries around stroke in the cortex support recovery of perfusion in the ischemic border region and support long-term recovery in rats (Wei, L. et al. , Stroke 32, 2179-84 (2001)). Further, it has been reported that some patients who did not experience immediate clinical amelioration by therapy, despite early arterial recanalization, showed delayed clinical amelioration (Alexandrov, A.V. et al. , Stroke 35, 449-52 (2004)). The authors of the report suppose recovery of the "stunned brain" by improvement in microenvironment is one of the possible mechanisms. In view of this, recovery of microenvironment in the peri-infarcted region may have some influence on the results, and the increase of arteries in HGF-treated rats may support recovery of cognitive function.

In clinical application to cerebral ischemia, timing for initiating gene therapy is important. Most of the previous reports have shown effects of gene therapy by injecting a gene of interest before the disorder or within several hours after the development of disorder. The purpose of those studies was to inhibit extension of the focus of apotosis and ischemia (Shirakura, M. et al. (2004), Supra, Hayashi, K. et al. (2001), Supra, Yoshimura et al. (2002), Supra, Zhang, W.R. et al. (2002), Supra). Different from studies made so far with regard to gene therapy, the target of the present inventors is to perform reconstruction of neuronal network and neoangiogenesis during the subacute stage to the chronic stage of ischemic disorder. What is important is that the present inventors could achieve recovery of functions by gene therapy at a later timing. In clinical application, it is impossible to administer a gene of interest within several hours, because it takes a long time to prepare a vector containing the gene of interest and to eliminate a malignant tumor prior to the gene therapy using a growth factor. Since "seven days" is a sufficient time period for preparing a vector and evaluating the general state of the tumor, it is recognized that the present studies demonstrated the possibility of gene therapy in clinical application. Further studies are required to clarify the best timing for gene therapy.

The results shown in Figure **11** shows the pssibility of HGF enhancing neurite outgrowth to produce new synaptogenesis. It thus revealed that amelioration of cerebral functions is actually supported by physical alteration of the nerve such as neurite outgrowth and synaptogenesis.

In conclusion, external application of HGF seven days after cerebral ischemia causes more preferable functional effects through the enhancement of growth of axon, neoangiogenesis and inhibition of gliosis in the peri-infarcted region. Although further studies are required for evaluating the safety of a vector and alternative administration route in clinical application, gene therapy using HGF may provide new therapeutic option in the treatment of cerebral ischemia.

### (Example 2: example of recovery of cerebral function by VEGF)

Next, experimentation of recovery of cerebral function by another cellular growth factor was performed. The experimentation was performed in the same manner as described in Example 1 except that VEGF (SEQ ID NO:4) was used instead of the HGF in Example 1. cDNA of human VEGF165 was cloned by an ordinary method (Science 246, 1306 (1989)), and was introduced into an expression vector, pUC-CAGGS.

As a result, amelioration of memory function and spatial learning function is similarly demonstrated.

### (Example 3: example of recovery of cerebral function by FGF)

Next, experimentation of recovery of cerebral function by yet another cellular growth factor was performed. The experimentation was performed in the same manner as described in Example 1 except that aFGF (SEQ ID NO:6) was used instead of the HGF in Example 1. The FGF is available from Kaken Pharmaceutical Co., Ltd.

As a result, amelioration of memory function and spatial learning function is similarly demonstrated.

Although the present invention has been illustrated hereinabove by way of preferred embodiments of the invention, the invention should not be understood as limited to the embodiments. It will be understood that the scope of the present invention is interpreted only depending on claims. From the specific description of preferred embodiments of the present invention, it will be understood that those skilled in the art can carry out the invention within an equivalent scope based on the description and common general knowledge. It will also be understood that a patent, patent application, and reference cited herein should be incorporated herein by reference of the present specification in its entirety as if the entirety per se were specifically described herein.

### INDUSTIAL APPLICABILITY

The present invention has industrial applicability in the pharmaceutical industry or the like where medicaments for amelioration or prevention of deterioration of cerebral functions are produced.

## Claims

1. A composition for prevention of deterioration of a cerebral function or amelioration of a cerebral function, the composition comprising a cellular growth factor as an active ingredient.

2. Thecompositionaccordingtoclaim 1, wherein the cellular growth factor has action of inducing vascular growth.

3. Thecompositionaccordingtoclaim 1, wherein the cellular growth factor is selected from the group consisting of: vascular endothelial growth factor (VEGF); fibroblast growth factor (FGF); and hepatocyte growth factor (HGF).

4. Thecompositionaccordingtoclaim 1, wherein the cellular growth factor is a hepatocyte growth factor (HGF).

5. Thecompositionaccordingtoclaim 1, wherein the cerebral function is a cognitive function or a motor function.

6. Thecompositionaccordingtoclaim 1, wherein the cerebral function is affected by cognitive dysfunction or motor dysfunction due to cerebral infarction, cerebral blood flow disorder, cerebral hemorrhage or cerebrovascular disorder.

7. Thecompositionaccordingtoclaim 1, wherein the cerebral function is selected from the group consisting of a memory function and a spatial learning function.

8. The composition according to claim 1, wherein prevention of deterioration of the cerebral function or amelioration of the cerebral function is achieved by activation of astrocytes.

9. Thecompositionaccordingtoclaim 1, wherein the cellular growth factor is provided in the form of a protein or in the form of a nucleic acid.

10. The composition according to claim 1, wherein the cellular growth factor is administered with a viral envelope.

11. The composition according to claim 10, wherein the viral envelope is inactivated.

12. The composition according to claim 10, wherein the viral envelope is an envelope of RNA virus.

13. The composition according to claim 10, wherein the viral envelope is an envelope of *Paramyxoviridae* virus.

14. The composition according to claim 10, wherein the viral envelope is an envelope of HVJ.

15. The composition according to claim 10, wherein the cellular growth factor is contained in the viral envelope.

16. The composition according to claim 1, which is delivered by administration to subarachnoid space or by intracisternal administration.

17. The composition according to claim 1, which is administered six days after the development of cerebral infarction, cerebral blood flow disorder, cerebral hemorrhage, cerebrovascular disorder or disorder of a cerebral function.

18. The composition according to claim 1, which is administered six days after the development of cerebral infarction, cerebral blood flow disorder, cerebral hemorrhage, cerebrovascular disorder or disorder of a cerebral function, wherein the cellular growth factor is in the form of a nucleic acid.

19. A method for prevention of deterioration of a cerebral function or amelioration of a cerebral function, the method comprising the step of:
(A) administering a cellular growth factor to a patient.

20. The method according to claim 19, wherein the cellular growth factor has action of inducing vascular growth.

21. The method according to claim 19, wherein the cellular growth factor is selected from the group consisting of: vascular endothelial growth factor (VEGF); fibroblast growth factor (FGF); and hepatocyte growth factor (HGF).

22. The method according to claim 19, wherein the cellular growth factor is a hepatocyte growth factor (HGF).

23. The method according to claim 19, wherein the cellular growth factor is administered with a viral envelope.

24. The method according to claim 23, wherein the viral envelope is an envelope of HVJ.

25. The method according to claim 19, wherein the cerebral function is a cognitive function or a motor function.

26. The method according to claim 19, wherein the cerebral function is affected by cognitive dysfunction or motor dysfunction due to cerebral infarction, cerebral blood flow disorder, cerebral hemorrhage or cerebrovascular disorder.

27. The method according to claim 19, wherein the cerebral function is selected from the group consisting of a memory function and a spatial learning function.

28. The method according to claim 19, wherein the cellular growth factor is in the form of a protein or in the form of a nucleic acid.

29. The method according to claim 19, wherein the cellular growth factor is delivered by administration to subarachnoid space or by intracisternal administration.

30. The method according to claim 19, wherein the cellular growth factor is administered six days after the development of cerebral infarction, cerebral blood flow disorder, cerebral hemorrhage, cerebrovascular disorder or disorder of a cerebral function.

31. A use of a cellular growth factor in the production of a medicament for prevention of deterioration of a cerebral function or amelioration of a cerebral function.

32. The use according to claim 31, wherein the cellular growth factor is selected from the group consisting of: vascular endothelial growth factor (VEGF); fibroblast growth factor (FGF); and hepatocyte growth factor (HGF).

33. The use according to claim 31, wherein the cellular growth factor is a hepatocyte growth factor (HGF).

34. The use according to claim 31, wherein the cellular growth factor is in the form of a protein or in the form of a nucleic acid.

35. A composition comprising a cellular growth factor used for enhancement of neurite outgrowth or synaptogenesis.

36. The composition according to claim 35, wherein the cellular growth factor is selected from the group consisting of: vascular endothelial growth factor (VEGF); fibroblast growth factor (FGF); and hepatocyte growth factor (HGF).

37. The composition according to claim 35, wherein the cellular growth factor is a hepatocyte growth factor (HGF).

38. The composition according to claim 35, wherein the cellular growth factor is in the form of a protein or in the form of a nucleic acid.

39. A method for enhancement of neurite outgrowth or synaptogenesis, the method comprising the step of:
(A) administering a cellular growth factor to an individual for which enhancement of the neurite outgrowth or synaptogenesis is necessary.

40. The method according to claim 39, wherein the cellular growth factor is selected from the group consisting of: vascular endothelial growth factor (VEGF); fibroblast growth factor (FGF); and hepatocyte growth factor (HGF).

41. The method according to claim 39, wherein the cellular growth factor is a hepatocyte growth factor (HGF).

42. The method according to claim 39, wherein the cellular growth factor is in the form of a protein or in the form of a nucleic acid.

43. A use of a cellular growth factor in the production of a medicament for enhancement of neurite outgrowth or synaptogenesis.

44. The use according to claim 43, wherein the cellular growth factor is selected from the group consisting of: vascular endothelial growth factor (VEGF); fibroblast growth factor (FGF); and hepatocyte growth factor (HGF).

45. The use according to claim 43, wherein the cellular growth factor is a hepatocyte growth factor (HGF).

46. The use according to claim 43, wherein the cellular growth factor is in the form of a protein or in the form of a nucleic acid.
